# EUROPÄISCHE PATENTSCHRIFT

(11) **EP 4 257 039 B1**
(45) Veröffentlichungstag und Bekanntmachung des Hinweises auf die Patenterteilung: **12.11.2025**
(21) Anmeldenummer: 23164882.5
(22) Anmeldetag: 29.03.2023
(51) Int. Cl.: A61B 5/08, A61B 5/097, A61B 5/00, G01N 33/497

(54) **ANALYSEGERÄT MIT EINER VERDRÄNGERPUMPE UND EINEM VENTIL UND ANALYSEVERFAHREN MIT EINEM SOLCHEN ANALYSEGERÄT**
ANALYTICAL DEVICE WITH A POSITIVE DISPLACEMENT PUMP AND A VALVE AND ANALYTICAL METHOD WITH SUCH AN ANALYTICAL DEVICE
ANALYSEUR AVEC UNE POMPE À DÉPLACEMENT POSITIF ET UNE SOUPAPE ET PROCÉDÉ D'ANALYSE UTILISANT UN TEL ANALYSEUR

(30) Priorität: 07.04.2022 DE 102022108432
(43) Veröffentlichungstag der Anmeldung: 11.10.2023
(73) Patentinhaber: Dräger Safety AG & Co. KGaA, 23560 Lübeck (DE)
(72) Erfinder: Reier, Tobias, 23558 Lübeck (DE); Brunswick Franco, Luis, 23558 Lübeck (DE); Bendorf, Nick, 23558 Lübeck (DE); Mattern-Fruehwald, Marie-Isabell, 23558 Lübeck (DE); Rekow, Jens, 23558 Lübeck (DE)
(74) Vertreter: Meyer-Gramann, Klaus Dieter

(56) Entgegenhaltungen:
- DE-U1- 202004 020 736
- US-A- 3 622 278
- US-A1- 2012 031 165

## Beschreibung

Die Erfindung betrifft ein Analysegerät und ein Verfahren zum Analysieren einer von einem Probanden abgegebenen Gasprobe, insbesondere einer ausgeatmeten Atemprobe, auf eine vorgegebene Substanz, insbesondere auf Alkohol. Ein solches Analysegerät lässt sich für die Überprüfung verwenden, ob der Proband Alkohol, insbesondere Äthylalkohol (Ethanol) und optional Beimischungen anderer Alkohole, zu sich genommen hat oder nicht. Wenn der Proband Alkohol oberhalb einer Nachweisgrenze zu sich genommen hat, so enthält sein Blut Alkohol und daher die vom Probanden ausgeatmete Luft Atemalkohol. Optional lässt sich mit einem solchen Analysegerät feststellen, wie groß die Atemalkohol-Konzentration in seinem Atem ist, und daraus lässt sich der Gehalt von Alkohol in seinem Blut ableiten.

Im Folgenden wird die Bezeichnung "Alkohol" für eine zu detektierende Substanz im Blut des Probanden verwendet, die Bezeichnung "Atemalkohol" für eine Substanz, die dann in einer Gasprobe, insbesondere Atemprobe, des Probanden enthalten ist, wenn sein Blut Alkohol enthält.

Verschiedene tragbare Analysegeräte für Atemalkohol sind bekannt geworden, beispielsweise aus DE 10 2017 008 008 A1. Ein Proband gibt eine Atemprobe in ein Mundstück des Analysegeräts ein. Wenigstens ein Teil der Atemprobe wird zu einer Messkammer im Analysegerät geleitet. Ein elektrochemischer Sensor misst, mit welcher Konzentration oder in welcher Menge das Gas, das von der Atemprobe stammt und in der Messkammer ist, Atemalkohol enthält. Natürlich kann die Messung zu dem Ergebnis führen, dass kein Atemalkohol oberhalb einer Nachweisgrenze in dem Gas und daher in der Atemprobe enthalten ist.

In DE 20 2024 020 736 U1 wird ein Prüfgerät für Atemalkohol beschrieben. Ein Proband gibt eine Atemprobe in ein an beiden Enden offenes Blasrohr 2 ein. Ein Eingangskanal 6 zweigt seitlich vom Blasrohr 2 ab und führt in eine Messkammer 8. Die Messkammer 8 befindet sich zwischen dem Blasrohr 2 und einer Fördervorrichtung 12 und ist mit der Fördervorrichtung 12 durch eine Leitung 14 verbunden. Die Fördervorrichtung 12 saugt eine Atemluftprobe aus dem Blasrohr 2 ein. Ein Kolben 16 mit einem magnetischen Kern 20 und einer Mantelfläche aus Glas kann sich in einem Zylinder 18 hin und her bewegen.

US 2012 / 0 031 165 A1 zeigt ein Prüfgerät für eine Atemprobe. Eine Pumpe 10 saugt eine Gasprobe durch eine Röhre 22 hindurch in eine Messkammer 20 hinein. Ein Kolben 12 der Pumpe 10 kann sich in einem Gehäuse 14 hin und her bewegen. Der Kolben 12 wird von einer Magnetspule 16 (solenoid) bewegt.

Ein Proband 10 gibt eine Atemprobe in ein Mundstück 12 des Prüfgeräts 14 von US 3 622 278 A ein. Die Atemprobe fließt durch ein Rückschlagventil 15 und erreicht ein Schaltventil 17 mit einem Einlass A und zwei Auslässen B und C. Der Auslass B ist mit einem Zylinder 19 verbunden, in dem sich ein Kolben 30 hin und her bewegen kann, so dass im Zylinder 19 eine Sammelkammer mit veränderlichem Volumen bereitgestellt wird. Der Ausgang C ist mit einem zweiten Schaltventil 32 verbunden. Das Schaltventil 32 hat einen Einlass D und zwei Auslässe E und F. Der Auslass E ist mit einer zweiten Sammelkammer verbunden, die sich in einem Zylinder 34 befindet, sowie mit einer Messkammer eines Sensors 16. Der Auslass F führt in die Umgebung. Dank der Schaltventile 17 und 32 wird nur der Teil der Gasprobe untersucht, der in die zweite Sammelkammer gelangt.

Der Erfindung liegt die Aufgabe zugrunde, ein Analysegerät und ein Verfahren zum Untersuchen einer Gasprobe auf eine vorgegebene Substanz bereitzustellen, wobei die Gasprobe von einem Probanden stammt, der auf eine vorgegebene Substanz zu untersuchen ist, und wobei das Analysegerät und das Verfahren zuverlässiger als bekannte Analysegeräte und Verfahren sein sollen.

Die Aufgabe wird durch ein Analysegerät mit den Merkmalen des Anspruchs 1 und durch ein Verfahren mit den Merkmalen des Anspruchs 11 gelöst. Vorteilhafte Ausgestaltungen des erfindungsgemäßen Analysegeräts sind, soweit sinnvoll, auch vorteilhafte Ausgestaltungen des erfindungsgemäßen Verfahrens und umgekehrt.

Das erfindungsgemäße Analysegerät und das erfindungsgemäße Verfahren vermögen eine Gasprobe auf eine vorgegebene Substanz zu untersuchen. Ein Proband hat diese Gasprobe abgegeben, insbesondere ausgeatmet. Die Substanz ist insbesondere Atemalkohol oder eine sonstige Droge oder sonstiges Suchtmittel, die / das sich in einer Gasprobe von einem Probanden nachweisen lässt.

Das Analysegerät umfasst eine Eingabeeinheit. Der Proband kann die Gasprobe in diese Eingabeeinheit hinein eingeben, insbesondere ausatmen, oder die Gasprobe wird auf andere Weise von der Eingabeeinheit aufgenommen. Die Eingabeeinheit ist dauerhaft oder wenigstens zeitweise mit dem Rest des Analysegerätes verbunden, bevorzugt lösbar verbunden.

Weiterhin umfasst das Analysegerät eine Messkammer sowie eine Eingabe-Fluidverbindung. Unter einer "Messkammer" wird ein Hohlraum des Analysegeräts verstanden, wobei der Hohlraum eine Gasprobe aufnehmen kann. Die Eingabe-Fluidverbindung verbindet wenigstens zeitweise die Eingabeeinheit mit der Messkammer, sodass bei hergestellter Fluidverbindung ein Fluid, insbesondere eine Menge der eingegebenen Gasprobe, von der Eingabeeinheit in die Messkammer hinein und bevorzugt umgekehrt aus der Messkammer heraus zurück in die Eingabeeinheit fließen kann.

Ein Sensor des Analysegeräts vermag ein Maß für die Menge und / oder die Konzentration der vorgegebenen Substanz in einem Gas zu messen. Zumindest vermag der Sensor festzustellen, ob die Menge oder Konzentration unterhalb oder oberhalb einer vorgegebenen Schranke liegt, z.B. einer Nachweisgrenze. Das Gas, welches der Sensor untersucht, befindet sich in oder an der Messkammer. Der Sensor ist bevorzugt in der Messkammer oder an oder in mindestens einer Wandung der Messkammer angeordnet. Der Sensor vermag ein Signal auszugeben, welches mit der Menge und / oder der Konzentration der Substanz korreliert.

Weiterhin umfasst das Analysegerät eine Ansaug-Kammereinheit, beispielsweise umfassend einen Balg und eine Platte, wobei die Platte den Balg zu dehnen und zu stauchen und dadurch das Volumen des vom Balgs umschlossenen Raums zu verändern vermag, oder mit einer Kolben-Zylinder-Einheit. Eine Ansaug-Fluidverbindung verbindet wenigstens zeitweise die Ansaug-Kammereinheit mit der Messkammer. Durch diese Ansaug-Fluidverbindung hindurch kann ein Fluid von der Messkammer in die Ansaug-Kammereinheit und zurück von der Ansaug-Kammereinheit in die Messkammer fließen.

Anmerkungen:
- Zwischen einem ersten Bauteil und einem zweiten Bauteil ist eine Fluidverbindung hergestellt, wenn ein Fluid, insbesondere ein Gas, vom ersten Bauteil in das zweite Bauteil fließen kann. Möglich ist, dass das erste Bauteil direkt an das zweite Bauteil anschließt und die Fluidverbindung mit Hilfe von zwei überlappenden Öffnungen in den beiden Bauteilen hergestellt ist.
- Möglich ist auch, dass die Fluidverbindung mithilfe einer Fluidführungseinheit hergestellt wird, wobei die Fluidführungseinheit die beiden Bauteile miteinander verbindet und ein Fluid durch die Fluidführungseinheit hindurch fließen kann. Unter einer "Fluidführungseinheit" wird ein Bauteil verstanden, welches ein Fluid entlang einer durch die Geometrie und Position des Bauteils vorgegebenen Trajektorie zu leiten vermag und idealerweise verhindert, dass das Fluid diese Trajektorie verlässt. Eine Röhre und ein Schlauch sind zwei Beispiele für eine Fluidführungseinheit.
- Das erfindungsgemäße Analysegerät stellt die Eingabe-Fluidverbindung und die Ansaug-Fluidverbindung jeweils wenigstens zeitweise während eines Einsatzes her. Möglich ist, dass in einem Ruhezustand des Analysegeräts keine der beiden Fluidverbindungen hergestellt ist.

Die Ansaug-Kammereinheit umschließt einen Raum, und zwar bis auf eine nachfolgend beschriebene Fluidverbindung fluiddicht, lässt sich wahlweise in einen Zustand mit minimalem Volumen oder in einen Zustand mit maximalem Volumen überführen. Bei der Überführung in den Zustand mit minimalem Volumen wird ein Fluid aus der Ansaug-Kammereinheit herausgefördert und in die Ansaug-Fluidverbindung hineingepresst. Dies bewirkt, dass Fluid aus der Ansaug-Fluidverbindung heraus in die Messkammer gepresst wird. Dadurch wird Fluid aus der Messkammer heraus gedrückt und gelangt in die Eingabe-Fluidverbindung oder in eine separate Ausgabe-Fluidverbindung. Der Vorgang, Fluid aus der Messkammer herauszudrücken, bewirkt daher, dass die Messkammer gespült wird. Insbesondere wird eine alte Gasprobe entfernt.

Bei der Überführung in den Zustand mit maximalem Volumen wird ein Fluid in die Ansaug-Kammereinheit hineingesaugt. Dies bewirkt, dass Fluid durch die Ansaug-Fluidverbindung hindurch in eine Kammer der Ansaug-Kammereinheit hineingesaugt wird. Dadurch wird ein Fluid aus der Umgebung durch die Eingabe-Fluidverbindung hindurch in die Messkammer gesaugt. Bevorzugt stammt wenigstens ein Teil dieses Fluids aus der Eingabeeinheit und enthält Gas, welches der Proband abgegeben hat. Der Vorgang, die Ansaug-Kammereinheit in den Zustand mit maximalem Volumen zu überführen, bewirkt also, dass wenigstens ein Teil der vom Probanden abgegebenen Gasprobe in die Messkammer gesaugt wird.

Das Analysegerät umfasst weiterhin ein Ventil. Dieses Ventil lässt sich wahlweise in eine verschließende Endposition oder in eine freigebende Endposition bewegen. In der verschließenden Endposition verschließt das Ventil die Eingabe-Fluidverbindung und unterbricht dadurch die Eingabe-Fluidverbindung. Selbstverständlich können auch bei verschlossener Eingabe-Fluidverbindung unvermeidliche Spalten oder Schlitze auftreten, durch die hindurch Gas aus der Eingabeeinheit in die Messkammer gelangen kann. In der freigebenden Endposition und optional auch in jeder Zwischenposition gibt das Ventil die Eingabe-Fluidverbindung vollständig oder wenigstens teilweise frei.

Weiterhin umfasst das Analysegerät eine Antriebseinheit. Bevorzugt umfasst die Antriebseinheit einen Stellantrieb und eine Stange.

Einerseits vermag die Antriebseinheit das Ventil wahlweise in die verschließende Endposition oder in die freigebende Endposition zu bewegen. Andererseits vermag dieselbe Antriebseinheit die Ansaug-Kammereinheit wahlweise in den Zustand mit maximalem Volumen oder in den Zustand mit minimalem Volumen zu überführen.

Die Antriebseinheit ist sowohl mit einem Verschlussteil, z.B. einem Ventilkörper, des Ventils als auch mit einem Teil der Ansaug-Kammereinheit mechanisch gekoppelt. Durch diese beiden Kopplungen wird eine der folgenden Wirkungen erzielt:
- Eine Bewegung des Ventils in die freigebende Endposition ist mit einer Überführung der Ansaug-Kammereinheit in den Zustand mit minimalem Volumen synchronisiert. Umgekehrt ist eine Bewegung des Ventils in die verschließende Endposition mit einer Überführung der Ansaug-Kammereinheit in den Zustand mit maximalem Volumen synchronisiert.
- Eine Bewegung des Ventils in die verschließende Endposition ist mit einer Überführung der Ansaug-Kammereinheit in den Zustand mit minimalem Volumen synchronisiert. Umgekehrt ist eine Bewegung des Ventils in die freigebende Endposition mit einer Überführung der Ansaug-Kammereinheit in den Zustand mit maximalem Volumen synchronisiert.

Das erfindungsgemäße Verfahren wird unter Verwendung eines erfindungsgemäßen Analysegeräts durchgeführt und umfasst die folgenden Schritte:
- Anfänglich ist das Ventil in der verschließenden Endposition und unterbricht die Eingabe-Fluidverbindung.
- Eine Gasprobe wird in die Eingabeeinheit eingegeben oder von der Eingabeeinheit aufgenommen.
- Die Antriebseinheit bewegt das Ventil in die freigebende Endposition, sodass das Ventil die Eingabe-Fluidverbindung freigibt.
- Die Antriebseinheit überführt die Ansaug-Kammereinheit in den Zustand mit maximalem Volumen. Dadurch wird Gas aus der Eingabeeinheit durch die Eingabe-Fluidverbindung hindurch in die Messkammer gesaugt.
- Anschließend überführt die Antriebseinheit die Ansaug-Kammereinheit wieder in den Zustand mit minimalem Volumen. Dadurch wird Gas aus der Ansaug-Kammereinheit durch die Ansaug-Fluidverbindung hindurch in die Messkammer gefördert. Dadurch wiederum wird Gas aus der Messkammer ausgestoßen, wodurch die Messkammer gespült wird.
- Die Antriebseinheit bewegt das Ventil wieder in die verschließende Endposition.

Die Reihenfolge, in der diese Schritte aufgelistet werden, ist nicht notwendigerweise diejenige zeitliche Reihenfolge, in der diese Schritte durchgeführt werden. Soweit sinnvoll, ist eine abweichende Reihenfolge ist möglich.

Außerdem umfasst das Verfahren den folgenden Schritt: Der Sensor misst ein Maß für die Konzentration und / oder die Menge der Substanz, insbesondere von Atemalkohol, in dem Gas, das sich in der Messkammer befindet. Wenigstens ein Teil dieses Gases in der Messkammer, idealerweise alles Gas in der Messkammer, stammt aus der Eingabeeinheit und daher vom Probanden.

In einer ersten Alternative der Erfindung werden die folgenden beiden Schritte gleichzeitig durchgeführt:
- Das Ventil wird in die freigebende Endposition bewegt.
- Die Ansaug-Kammereinheit wird in den Zustand mit minimalem Volumen überführt.

Gemäß der ersten Alternative werden auch die folgenden beiden Schritte gleichzeitig durchgeführt:
- Das Ventil wird in die verschließende Endposition bewegt.
- Die Ansaug-Kammereinheit wird in den Zustand mit maximalem Volumen überführt.

In einer zweiten Alternative der Erfindung werden die folgenden beiden Schritte gleichzeitig durchgeführt:
- Das Ventil wird in die verschließende Endposition bewegt.
- Die Ansaug-Kammereinheit wird in den Zustand mit minimalem Volumen überführt.

Gemäß der zweiten Alternative werden auch die folgenden beiden Schritte gleichzeitig durchgeführt:
- Das Ventil wird in die freigebende Endposition bewegt.
- Die Ansaug-Kammereinheit wird in den Zustand mit maximalem Volumen überführt.

Das Merkmal, dass zwei Vorgänge gleichzeitig ausgeführt werden, schließt ein, dass zwischen den Anfangs-Zeitpunkten oder End-Zeitpunkten der beiden Vorgänge jeweils ein Unterschied innerhalb eines Toleranzbandes auftritt.

Durch das erfindungsgemäße Analysegerät und das erfindungsgemäße Verfahren wird mindestens eine Gasprobe aus der Eingabeeinheit in die Messkammer gesaugt. Diese Gasprobe ist ein Teil derjenigen Menge von Gas, welche der Proband in die Eingabeeinheit eingegeben hat. Wie bereits dargelegt, wird die Gasprobe in die Messkammer gesaugt, indem die Ansaug-Kammereinheit in den Zustand mit maximalem Volumen überführt wird. Bevorzugt ist die Eingabeeinheit wenigstens in dem Zeitraum, in dem die Gasprobe in die Messkammer gesaugt wird, mit dem Rest des Analysegerätes verbunden, so dass sichergestellt ist, dass die eingesogene Gasprobe von dem Gas stammt, welches der Proband in die Eingabeeinheit ausgeatmet oder auf andere Weise eingegeben hat.

Die zu untersuchende Gasprobe kann natürlich nur dann aus der Eingabeeinheit in die Messkammer fließen, um dort von dem Sensor untersucht zu werden, wenn das Ventil in der freigebenden Endposition oder wenigstens in einer Zwischenposition ist. Außerdem kann in vielen Fällen nur dann die Messkammer gespült und für die Aufnahme einer weiteren Gasprobe verfügbar gemacht werden, wenn das Ventil in der freigebenden Endposition oder wenigstens in einer Zwischenposition ist. Nur dann kann Gas aus der Messkammer durch die Eingabe-Fluidverbindung hindurch aus der Messkammer heraus fließen. Möglich ist auch, dass Gas aus der Messkammer nicht durch die Eingabe-Fluidverbindung hindurch, sondern durch eine separate Fluidverbindung das Analysegerät verlässt.

Wenn das Ventil in der verschließenden Endposition ist, so ist die Messkammer von der Eingabeeinheit und bevorzugt von der Umgebung getrennt, idealerweise fluiddicht getrennt. Dadurch ist einerseits die Gefahr geringer, dass Umgebungsbedingungen den Sensor verändern. Insbesondere wird die Gefahr verringert, dass Partikel oder Substanzen aus der Eingabeeinheit oder der Umgebung auf den Sensor einwirken oder sich Ablagerungen bilden. Andererseits ist die Gefahr geringer, dass ein Bestandteil des Sensors verdunstet, beispielsweise ein Elektrolyt. Sowohl Umgebungsbedingungen als auch ein Verdunsten können dazu führen, dass der Sensor falsche oder unzuverlässige Messergebnisse liefert oder gar ausfällt.

Aus den gerade genannten Gründen muss das Ventil zeitweise in der freigebenden Position sein, nämlich damit eine Probe der zu untersuchenden Gasprobe in die Messkammer fließen kann, und sollte ansonsten in der verschließenden Position sein.

Erfindungsgemäß saugt die Ansaug-Kammereinheit dadurch eine Probe der zu untersuchenden Gasprobe in die Messkammer, dass die Ansaug-Kammereinheit in den Zustand mit maximalem Volumen überführt wird. Aufgrund dieses Merkmals wird in vielen Fällen eine definierte Menge der zu untersuchenden Gasprobe in relativ kurzer Zeit in die Messkammer gefördert. Die Menge der Gasprobe, die in die Messkammer gelangt, ließe sich in vielen Fällen viel schwieriger bestimmen, wenn die Menge wesentlich von der Stärke und der Dauer, mit der der Proband die Gasprobe eingibt, abhängen würde, sowie davon, mit welchem Volumenfluss die Gasprobe in die Messkammer fließt, oder wenn die Gasprobe in die Messkammer diffundieren würde. Außerdem erfordert ein solches Vorgehen, um eine Gasprobe in die Messkammer zu verbringen, in vielen Fällen mehr Zeit als die erfindungsgemäße Verwendung der Ansaug-Kammereinheit.

In vielen Fällen befindet sich in der Messkammer fast nur dasjenige Gas, das dadurch in die Messkammer gesaugt worden ist, dass die Ansaug-Kammereinheit in den Zustand mit maximalem Volumen überführt worden ist. Das Gas, welches durch Diffundieren oder dadurch, dass der Proband bei ruhender Ansaug-Kammereinheit eine Gasprobe ausstößt, in die Messkammer gelangt, hat oft eine vernachlässigbar kleine Menge. Die Wirkung, dass das Gas in der Messkammer fast nur vom Ansaugen stammt, erleichtert es in vielen Fällen sicherzustellen, dass im Wesentlichen nur Luft aus der Lunge des Probanden in die Messkammer gelangt, aber nur wenig Luft aus den oberen Atemwegen oder aus dem Mund. Dies erhöht insbesondere dann, wenn Alkohol im Blut des Probanden detektiert werden soll, die Zuverlässigkeit der Messung. In einer anderen Anwendung erleichtert es dieses Merkmal sicherzustellen, dass zunächst Luft aus dem Mund und anschließend Luft aus der Lunge des Probanden in die Messkammer gelangt, also durch zwei Ansaugvorgänge, und dort jeweils auf die Substanz untersucht wird, während weitgehend verhindert wird, dass Luft aus den oberen Atemwegen in die Messkammer gelangt.

Dank der Erfindung wird nur eine einzige Antriebseinheit benötigt, um sowohl das Ventil zu bewegen als auch die Ansaug-Kammereinheit zu überführen. Dadurch werden eine Antriebseinheit und damit in vielen Fällen Platz und elektrische Energie eingespart im Vergleich zu einer Ausgestaltung, bei der zwei verschiedene Antriebseinheiten vorgesehen sind. Außerdem ist nur eine Antriebseinheit mit elektrischer Energie zu versorgen, anzusteuern und zu überwachen, nicht aber zwei.

Erfindungsgemäß ist die Bewegung des Ventils von der einen Endposition in die andere Endposition mit der Überführung der Ansaug-Kammereinheit von dem einen Zustand in den anderen Zustand mechanisch gekoppelt, und zwar dadurch, dass dieselbe Antriebseinheit sowohl mit dem Ventil als auch mit der Ansaug-Kammereinheit mechanisch gekoppelt ist. Dank dieser Kopplung wird in vielen Fällen erreicht, dass das Ventil
- nur so lange wie nötig geöffnet ist, nämlich damit Gas in die Messkammer fließen kann, und
- so lange wie möglich geschlossen ist und dadurch die Messkammer und den Sensor von der Umgebung und von der Eingabeeinheit abtrennt.

Dank der mechanischen Kopplung braucht keine elektronische oder pneumatische Steuerung oder Regelung verwendet werden, welche die VentilBewegung mit der Zustands-Überführung koppelt.

Erfindungsgemäß verbindet die Eingabe-Fluidverbindung die Eingabeeinheit mit der Messkammer. In einer Realisierungsform umfasst oder definiert die Eingabeeinheit einen Kanal, der die Umgebung mit der Eingabe-Fluidverbindung verbindet. Bevorzugt verjüngt dieser Kanal sich gesehen in eine Richtung auf die Messkammer zu. In einer Realisierungsform umfasst die Eingabeeinheit weiterhin ein Mundstück, welches sich lösbar mit einem Gehäuse des Analysegeräts verbinden lässt und eine abgegebene Gasprobe, insbesondere eine Atemprobe, in Richtung der Eingabe-Fluidverbindung leitet. Diese beiden Realisierungsformen lassen sich miteinander kombinieren.

Erfindungsgemäß vermag die Antriebseinheit sowohl das Ventil zu bewegen als auch die Ansaug-Kammereinheit zu überführen und dadurch eine synchronisierte Bewegung dieser beiden Teile hervorzurufen. Bevorzugt umfasst die Antriebseinheit einen Stellantrieb, der sowohl mit dem Ventil als auch mit der Ansaug-Kammereinheit mechanisch gekoppelt ist.

Erfindungsgemäß bewirkt der Schritt, dass die Ansaug-Kammereinheit in den Zustand mit maximalem Volumen überführt wird, folgendes: Gas aus der Eingabeeinheit wird durch die Eingabe-Fluidverbindungen hindurch in die Messkammer gesaugt. Bevorzugt befindet die Messkammer sich zwischen der Eingabeeinheit und Ventil auf der einen Seite und der Ansaugkammer-Einheit auf der anderen Seite. Besonders bevorzugt sind die Eingabeeinheit, die Eingabe-Fluidverbindung, die Messkammer und die Ansaug-Kammereinheit hintereinander entlang einer Linie angeordnet. Bevorzugt befindet das Ventil sich ebenfalls auf dieser Linie, und zwar zwischen der Eingabeeinheit und der Messkammer. Die Ausgestaltung mit der Messkammer zwischen der Eingabeeinheit und der Ansaug-Kammereinheit führt zu einer besonders platzsparenden und robusten Anordnung. Die bevorzugte Ausgestaltung, bei der verschiedene Bestandteile entlang einer Linie angeordnet sind, bewirkt in vielen Fällen folgendes: Die Gasprobe wird linear gefördert, was die Gefahr von unerwünschten Verwirbelungen reduziert.

In einer bevorzugten Realisierungsform umfasst die Antriebseinheit zusätzlich ein mechanisches Ventil-Verbindungselement. Das Ventil-Verbindungselement ist oder umfasst bevorzugt einen Stab. Das Ventil umfasst ein Verschlussteil, das bevorzugt als Ventilkörper ausgestaltet ist, sowie einen Verschlussteil-Sitz, beispielsweise einen Dichtungsring. Das Verschlussteil ist relativ zum Verschlussteil-Sitz beweglich, bevorzugt linear in zwei entgegengesetzte Richtungen beweglich. Wenn das Ventil sich in der verschließenden Endposition befindet, liegt das Verschlussteil am Verschlussteil-Sitz an, bevorzugt fluiddicht bis auf unvermeidliche Schlitze und / oder Spalten. Wenn das Ventil sich in der freigebenden Endposition oder in einer Zwischenposition befindet, tritt ein Abstand zwischen dem Verschlussteil und dem Verschlussteil-Sitz auf, in einer Realisierungsform ein ringförmiger Spalt. Gemäß dieser Realisierungsform verbindet das Ventil-Verbindungselement den Stellantrieb mechanisch mit dem Verschlussteil. Diese Realisierungsform führt zu einem besonders einfachen mechanischen Aufbau. Das Ventil-Verbindungselement überbrückt den Abstand zwischen dem Stellantrieb und dem Verschlussteil. Dadurch lässt der Stellantrieb sich räumlich entfernt vom Verschlussteil anordnen, was in manchen Fällen die Positionierung der Eingabe-Fluidverbindung sowie die elektrische Versorgung des Stellantriebs erleichtert. Eine mögliche Erwärmung des Stellantriebes wirkt sich in vielen Fällen weniger auf andere Teile des analysegeräts aus.

Diese Ausgestaltung lässt sich mit einer Ausgestaltung kombinieren, bei der sich die Ansaug-Kammereinheit zwischen der Messkammer und dem Stellantrieb befindet. Bevorzugt befindet sich die Messkammer wiederum zwischen der Ansaug-Kammereinheit und der Eingabeeinheit.

In einer weiteren Ausgestaltung umfasst die Ansaug-Kammereinheit eine Ansaug-Kammer mit veränderbarem Volumen und einer fluiddichten Wandung, beispielsweise einen Balg. Außerdem umfasst die Ansaug-Kammereinheit ein mechanisches Kammer-Veränderungselement, beispielsweise eine Platte. Möglich ist auch, dass die Ansaug-Kammereinheit eine Kolben-Zylinder-Einheit umfasst, wobei der Kolben relativ zum Zylinder beweglich ist und die Ansaug-Kammer im Zylinder gebildet und vom Kolben begrenzt wird. Der Kolben fungiert dann als das Kammer-Veränderungselement. Eine Bewegung des Kammer-Veränderungselements relativ zu der Ansaug-Kammer bewirkt, dass das Volumen der Ansaug-Kammer verändert wird. Die erfindungsgemäße Ansaug-Fluidverbindung verbindet die Ansaug-Kammer mit der Messkammer. Die Antriebseinheit umfasst einen Stellantrieb und ein mechanisches Kammer-Verbindungselement. Dieses Kammer-Verbindungselement verbindet den Stellantrieb mit dem Kammer-Veränderungselement.

Mindestens zwei der gerade genannten Ausgestaltungen lassen sich miteinander kombinieren.

Erfindungsgemäß vermag die Antriebseinheit das Ventil zwischen der freigebenden und der verschließenden Endposition hin und her zu bewegen. In einer Ausgestaltung vermag ein Volumenfluss-Sensor ein Maß für den Volumenfluss von Gas durch die Eingabe-Fluidverbindung hindurch in die Messkammer zu messen. Unter einem "Volumenfluss" durch eine Fluidverbindung wird ein Volumen pro Zeiteinheit eines Fluids verstanden, welches durch die Fluidverbindung fließt. Beispielsweise misst der Volumenfluss-Sensor den Unterschied des Drucks an zwei verschiedenen Messpositionen, und eine Auswerteeinheit leitet aus dem Druck-Unterschied den Volumenfluss her. Gemäß dieser Ausgestaltung ist das erfindungsgemäße Analysegerät wie folgt ausgestaltet: Abhängig vom gemessenen Volumenfluss vermag ein Steuergerät des Analysegeräts automatisch den Schritt auszulösen, dass die Antriebseinheit das Ventil in die verschließenden Endposition bewegt. Diese Ausgestaltung erleichtert es, eine definierte und / oder bekannte Menge des Gases in die Messkammer zu leiten und danach die Messkammer zu verschließen. Diese Ausgestaltung steigert die Zuverlässigkeit des Messergebnisses. Die Menge des Gases lässt sich aus dem gemessenen Volumenfluss herleiten.

Erfindungsgemäß verbindet die Eingabe-Fluidverbindung die Eingabeeinheit mit der Messkammer. In der freigebenden Endposition gibt das Ventil die Eingabe-Fluidverbindung frei. In einer Ausgestaltung umgibt eine Fluidführungseinheit, beispielsweise eine Röhre, ein Verschlussteil, z.B. einen Ventilkörper, des Ventils und optional auch einen Teil eines Ventil-Verbindungselements der Antriebseinheit. Zwischen der Fluidführungseinheit und dem Verschlussteil tritt ein Zwischenraum auf, beispielsweise ein ringförmiger Spalt. Die Eingabe-Fluidverbindung führt durch die Fluidführungseinheit hindurch und umfasst diesem Zwischenraum. In einer Ausgestaltung grenzt ein Ventilkörper-Sitz des Ventils an diesen Zwischenraum an.

Diese Ausgestaltung ermöglicht es in besonders einfacher Weise, das Ventil so auszugestalten, dass eine lineare Bewegung des Verschlussteils (z. B. des Ventilkörpers) das Ventil von der einen Endposition in die andere Endposition bewegt. Die Fluidführungseinheit schützt bis zu einem gewissen Grad das Verschlussteil vor mechanischen Beschädigungen von außen.

Erfindungsgemäß bewirkt der Schritt, die Ansaug-Kammereinheit in den Zustand mit maximalem Volumen zu überführen, dass Gas durch die Eingabe-Fluidverbindung hindurch in die Messkammer gesaugt wird. In einer Ausgestaltung bewirkt der Schritt, die Ansaug-Kammereinheit in den Zustand mit minimalem Volumen zu überführen, dass Gas durch die Eingabe-Fluidverbindung hindurch aus der Messkammer hinaus gefördert wird, beispielsweise ausgestoßen wird. Dadurch wird die Messkammer gespült und vermag eine neue Gasprobe aufzunehmen. Möglich, aber nicht erforderlich ist eine separate Fluidverbindung, um die Messkammer zu spülen.

In einer bevorzugten Ausgestaltung löst ein Steuergerät des Analysegeräts den Schritt, das Ventil in die freigebende Endposition zu überführen und dadurch die Eingabe-Fluidverbindung freizugeben, automatisch und zeitgesteuert oder ereignisgesteuert ausgelöst. Gemäß einer Realisierungsform wird das Ereignis detektiert, dass die Eingabe der Gasprobe in die Eingabeeinheit begonnen wird. Beispielsweise detektiert ein Sensor das Ereignis, dass ein Mundstück oder ein sonstiges Eingabeelement auf einen Grundkörper des Analysegeräts aufgesetzt worden ist oder dass Gas in das Eingabeelement fließt.

Der Schritt, das Ventil in die freigebende Endposition zu bewegen, wird in einer ersten Realisierungsform begonnen, wenn seit dem Ereignis, dass die Eingabe oder Aufnahme der Gasprobe begonnen worden ist, eine vorgegebene Zeitspanne verstrichen ist.

In einer zweiten Realisierungsform wird der Schritt, das Ventil in die freigebende Endposition zu überführen, begonnen, wenn seit dem Beginn der Gas-Eingabe ein vordefiniertes öffnendes Ereignis eingetreten ist. Das öffnende Ereignis hängt bevorzugt davon ab, welches Volumen oder welche Menge die Gasprobe hat, die bislang in die Eingabeeinheit eingegeben wurde und / oder in die Eingabeeinheit hineinfließt.

Beide Realisierungsformen der bevorzugten Ausgestaltung tragen in einer Anwendung der Erfindung dazu bei, dass im Wesentlichen nur Luft aus der Lunge des Probanden in die Messkammer fließt, aber überhaupt nicht oder nur wenig Luft aus den oberen Atemwegen und dem Mund. Diese Wirkung erhöht die Zuverlässigkeit, durch eine Analyse der Gasprobe auf Atemalkohol den Gehalt von Alkohol im Blut des Probanden korrekt zu ermitteln. In einer anderen Anwendung der Erfindung tragen die Realisierungsformen dabei, das zunächst im wesentlichen Luft aus dem Mund und anschließend im wesentlichen Luft aus der Lunge in die Messkammer fließt, aber keine Luft aus den oberen Atemwegen.

Gas kann im Wesentlichen nur dann in die Messkammer gelangen, wenn das Ventil in der freigebenden Endposition ist. In der Regel ist das Ventil nur für sehr kurze Zeit in einer Zwischenposition zwischen den beiden Endpositionen. In einer Ausgestaltung wird mit höherer Zuverlässigkeit sichergestellt, dass die Menge des Gases in der Messkammer wenigstens annähernd bekannt ist. In einer Ausgestaltung wird gemessen, welche Menge von Gas bislang in die Messkammer geflossen ist. Wenn die gemessene Menge eine vorgegebene Mengen-Schranke erreicht, so wird der Schritt ausgelöst, das Ventil wieder in die verschließende Endposition zu bewegen.

Erfindungsgemäß überführt die Antriebseinheit die Ansaug-Kammereinheit in den Zustand mit maximalem Volumen und in den Zustand mit minimalem Volumen. Alternative Ausgestaltungen sind möglich, in welcher Reihenfolge diese beiden Schritte durchgeführt werden.

In einer ersten Alternative ist die Ansaug-Kammereinheit im Zustand mit maximalem Volumen, bevor das Verfahren durchgeführt wird, also bevor eine zu untersuchende Gasprobe in die Messkammer gelangt. Zuerst wird der Schritt durchgeführt, die Ansaug-Kammereinheit in den Zustand mit minimalem Volumen zu überführen. Dadurch wird die Messkammer gespült, wobei insbesondere Gas von einer früheren Gasprobe aus der Messkammer entfernt wird. Anschließend wird der Schritt durchgeführt, die Ansaug-Kammereinheit wieder in den Zustand mit maximalem Volumen zu überführen. Dadurch wird eine Menge der aktuell zu untersuchenden Gasprobe in die Messkammer gesaugt.

Der Sensor misst nunmehr ein Maß für die Menge oder die Konzentration der Substanz.

In einer zweiten Alternative wird die umgekehrte Reihenfolge durchgeführt. Bevor das Verfahren durchgeführt wird, ist die Ansaug-Kammereinheit im Zustand mit minimalem Volumen. Zuerst wird der Schritt durchgeführt, die Ansaug-Kammereinheit in den Zustand mit maximalem Volumen zu überführen. Dadurch wird eine Menge der aktuell zu untersuchenden Gasprobe in die Messkammer gesaugt. Der Sensor misst ein Maß für die Menge oder die Konzentration der Substanz. Anschließend wird die Ansaug-Kammereinheit wieder in den Zustand mit minimalem Volumen überführt. Dadurch wird die Messkammer gespült.

In einer bevorzugten Realisierungsform umfasst das Analysegerät eine Eingabe-Fluidführungseinheit. Die Eingabe-Fluidverbindung ist durch die Eingabe-Fluidführungseinheit hindurchgeführt. Ein Teil der Eingabe-Fluidführungseinheit kann zu einer Wandung der Messkammer gehören. Die Eingabeeinheit lässt sich mit der Eingabe-Fluidführungseinheit verbinden, bevorzugt lösbar verbinden, verbinden, beispielsweise auf eine Röhre aufsetzen. Die Eingabe-Fluidführungseinheit umgibt das Ventil, bevorzugt vollständig. Diese Realisierungsform führt zu einer besonders platzsparenden Ausgestaltung des Analysegeräts und erfordert weniger Bauraum als eine andere mögliche Anordnung des Ventils. Außerdem führt diese Realisierungsform in vielen Fällen zu einer besonders robusten Ausgestaltung, und die Gefahr wird reduziert, dass das Ventil durch Einflüsse von außen beschädigt oder undicht wird.

In einer ersten Anwendung wird einmal eine Gasprobe, die der Proband abgegeben hat, aus der Eingabeeinheit in die Messkammer gesaugt und dort vom Sensor analysiert. Wie bereits oben dargelegt, erleichterte es die Erfindung sicherzustellen, dass diese Gasprobe überwiegend aus Luft aus der Lunge des Probanden besteht, aber nur zu geringen Teil Luft aus den oberen Atemwegen und aus dem Mund des Probanden umfasst. Bei dieser ersten Anwendung soll der Gehalt der Substanz im Blut des Probanden untersucht werden.

In einer zweiten Anwendung werden nacheinander zwei Gasproben, die von demselben Probanden eingegeben wurden, aus der Eingabeeinheit in die Messkammer gesaugt und dort vom Sensor analysiert. Zwischendurch wird die Messkammer ausgespült. Bei dieser zweiten Anwendung wird das Analysegerät so betrieben, dass die erste Gasprobe im Wesentlichen aus Luft aus dem Mund des Probanden besteht, die zweite Gasprobe im Wesentlichen aus Luft aus der Lunge. Mit Hilfe der ersten Gasprobe wird untersucht, ob der Proband vor kurzem Alkohol oder eine sonstige Substanz zugenommen zu sich genommen hat, wobei diese Substanz noch nicht den Blutkreislauf des Probanden erreicht hat. Mit der zweiten Gasprobe wird wiederum der Gehalt der Substanz im Blut des Probanden untersucht.

Bei einer dritten Anwendung wird zunächst eine Vorprobe in die Messkammer gesaugt, und mit dieser Vorprobe wird die Messkammer ausgespült, ohne dass notwendigerweise die Vorprobe auf die Substanz untersucht wird. Die Vorprobe kann ebenfalls vom Probanden oder auch aus der Umgebung stammen.

In einer Ausgestaltung ist das Analysegerät als ein tragbares Gerät ausgestaltet und umfasst eine eigene Spannungsversorgungseinheit, bevorzugt einen Satz von wiederaufladbaren Batterien. Das Analysegerät kann auch als stationäres Gerät ausgestaltet und an ein stationäres Energieversorgungsnetz angeschlossen wird, oder anschließbar sein.

Im Folgenden wird die Erfindung anhand eines Ausführungsbeispiels beschrieben. Hierbei zeigt
- Figur 1: schematisch die Wirkungsweise eines elektrochemischen Sensors;
- Figur 2: in einer perspektivischen Darstellung schräg von oben eine erste Ausgestaltung des erfindungsgemäßen Analysegeräts;
- Figur 3: in einer perspektivischen Darstellung senkrecht von oben das Analysegerät von Figur 2;
- Figur 4: in einer Querschnittsdarstellung das Analysegerät von Figur 2 und Figur 3;
- Figur 5: in einer weiteren Querschnittsdarstellung das Analysegerät von Figur 2 und Figur 3;
- Figur 6: in einer perspektivischen Darstellung ein Segment des Analysegeräts gemäß der ersten Ausgestaltung umfassend den Probeneinlass, das Ventil, die Stange, den Sensor und den Balg, wobei die Messkammer fortgelassen ist;
- Figur 7: in einer Querschnittsdarstellung das Segment von Figur 6, wobei der Sensor fortgelassen ist;
- Figur 8: schematisch in einer Querschnittsdarstellung die Eingabe-Fluidverbindung zur Messkammer;
- Figur 9: in einer Querschnittsdarstellung eine zweite Ausgestaltung des erfindungsgemäßen Analysegeräts;
- Figur 10: drei Schnitte durch das Analysegerät, wobei die Stange senkrecht auf den Zeichenebenen steht.

Im Ausführungsbeispiel wird das erfindungsgemäße Analysegerät dafür verwendet, um eine Atemprobe, die ein Proband ausgeatmet hat, auf eine vorgegebene Substanz, insbesondere auf Atemalkohol, zu analysieren. Im Falle von Atemalkohol als der Substanz soll ein Proband daraufhin untersucht werden, ob sich in seinem Blut Alkohol oberhalb einer Nachweisgrenze befindet oder nicht. Der Proband gibt eine Atemprobe in ein Mundstück des Analysegeräts ein. Falls der Proband Alkohol zu sich genommen hat und der Alkohol im Blut noch nicht vollständig abgebaut ist, so enthält die abgegebene Atemprobe Atemalkohol. Ein Teil der abgegebenen Atemprobe fließt in eine Messkammer im Inneren des Analysegeräts. Dieser Teil wird im Folgenden als "Messkammer-Probe" bezeichnet. Ein Sensor in oder an der Messkammer prüft, ob diese Messkammer-Probe Atemalkohol oder eine sonstige vorgegebene Substanz enthält oder nicht. Die Erfindung lässt sich auch für eine andere Substanz verwenden, die in der ausgeatmeten Luft eines Probanden oder in einem sonstigen Gas, das ein Proband abgeben kann, enthalten sein kann.

Der Sensor vermag ein Signal zu erzeugen, welches mit der Menge und / oder Konzentration der vorgegebenen Substanz in der Messkammer-Probe, welche in der Messkammer ist, korreliert. Aus dem Stand der Technik sind unterschiedliche geeignete Sensoren bekannt, beispielsweise elektrochemische Sensoren, photooptische Sensoren, photo-akustische Sensoren, Photo-Ionisierungs-Sensoren und Wärmetönungssensoren. Ein solcher Sensor lässt sich auch für die Erfindung anwenden.

Das Analysegerät leitet aus der gemessenen Menge oder Konzentration von Atemalkohol in der Messkammer-Probe sowie aus der Menge und / oder dem Volumen der Messkammer-Probe die Konzentration von Atemalkohol in der eingegebenen Atemprobe ab. Die Menge und / oder das Volumen der Messkammer-Probe wird beispielsweise abhängig von dem Volumen der Messkammer, welches durch die Konstruktion des Analysegeräts bekannt ist, und / oder dadurch, dass der Volumenfluss in die Messkammer mehrfach gemessen wird und die Messwerte aufintegriert werden, hergeleitet. Falls die Substanz Atemalkohol ist, so leitet eine signalverarbeitende Auswerteeinheit des Analysegeräts oder eine räumlich entfernte Auswerteeinheit aus der Atemalkohol-Konzentration in der Atemprobe den aktuellen Gehalt von Alkohol im Blut des Probanden ab.

Während die Atemprobe durch das Mundstück hindurch fließt, fließt zunächst Luft aus dem Mund, dann aus den oberen Atemwegen und anschließend Luft aus der Lunge des Probanden durch das Mundstück. Um festzustellen, ob das Blut des Probanden Alkohol enthält, ist ein Gas aus demjenigen Teil der Atemprobe zu untersuchen, der aus der Lunge stammt. Idealerweise fließt nur ein Gas, welches aus der Lunge des Probanden stammt, in die Messkammer, und die Messkammer-Probe enthält nur Luft aus der Lunge, aber keine Luft aus dem Mund und den oberen Atemwegen. Nachfolgend wird beschrieben, wie dieses Ziel erfindungsgemäß erreicht wird.

Das Analysegerät des Ausführungsbeispiels umfasst einen elektrochemischen Sensor 12. Unter einem "Sensor" wird ein Bauteil verstanden, welches automatisch ein Signal generiert, bevorzugt ein elektrisches Signal, wobei das generierte Signal ein Maß für die Menge und / oder Konzentration einer vorgegebenen Substanz in der Messkammer-Probe ist. Diese Messkammer-Probe befindet sich in einer Messkammer, wobei der Sensor diese Messkammer-Probe in der Messkammer zu analysieren vermag. Ein elektrochemischer Sensor löst eine chemische Reaktion aus, wobei die chemische Reaktion von der Menge und / oder Konzentration der zu analysierenden Substanz abhängt und eine messbare elektrische Detektions-Größe, beispielsweise die Stromstärke oder die elektrische Spannung oder die elektrische Ladung oder den elektrischen Widerstand eines Bauteils des Sensors, beeinflusst.

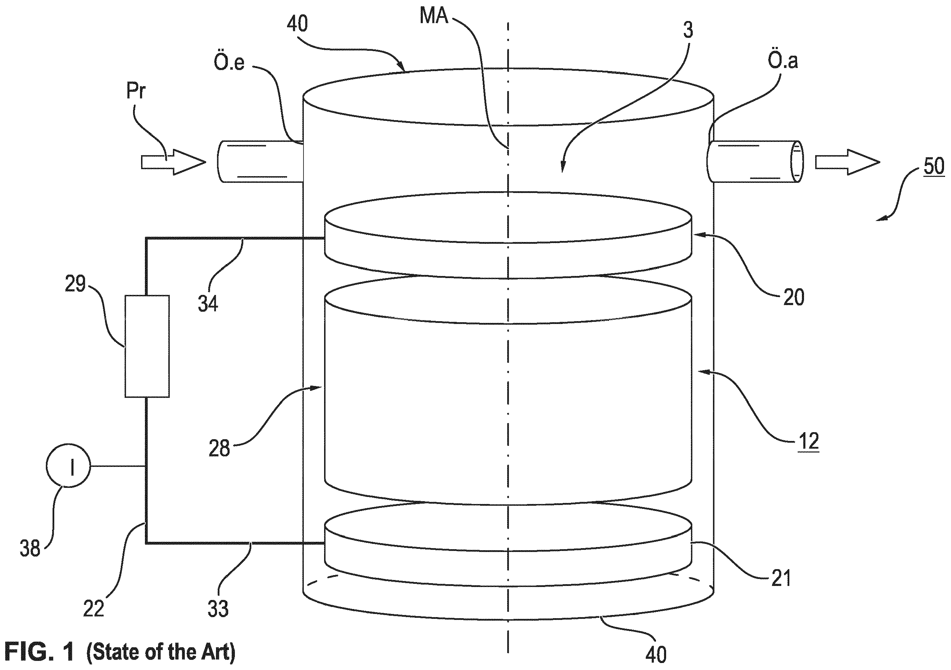

Figur 1 zeigt schematisch und beispielhaft die Wirkungsweise eines elektrochemischen Sensors 12, wie er aus dem Stand der Technik bekannt ist. Die Darstellung von Figur 1 ist nicht notwendigerweise maßstabsgerecht. Dieser elektrochemische Sensor 12 vermag eine Messkammer-Probe Pr auf Atemalkohol zu analysieren und arbeitet nach dem Prinzip einer Brennstoffzelle mit Alkohol als dem Brennstoff. Ein solcher Sensor 12 lässt sich auch für das erfindungsgemäße Analysegerät verwenden. Das erfindungsgemäße Analysegerät umfasst in einer Ausgestaltung einen derartigen elektrochemischen Sensor 12.

Mit dem Bezugszeichen 50 wird in Figur 1 eine Sensor-Anordnung bezeichnet, welche einen elektrochemischen Sensor 12 und eine Wandung 40 für eine Messkammer 3 umfasst. Die Wandung 40 umgibt den Sensor 12 und die Messkammer 3. In der gezeigten Realisierungsform sind sowohl die Wandung 40 als auch der Sensor 12 rotationssymmetrisch zu derselben Mittelachse MA. Selbstverständlich sind auch andere geometrische Formen möglich.

Die zu analysierende Messkammer-Probe Pr, die im Ausführungsbeispiel von einer Atemprobe A stammt, fließt durch eine eintrittsseitige Öffnung Ö.e hindurch in das Innere der Messkammer 3, z.B. indem sie von einem Probanden ausgeatmet oder angesaugt wird oder in die Messkammer 3 diffundiert. In einer Ausgestaltung fließt die Messkammer-Probe Pr durch eine austrittseitige Öffnung Ö.a wieder aus der Messkammer 3 heraus. Dank dieser Ausgestaltung kann der Sensor 12 rasch nacheinander mehrere Messkammer-Proben Pr untersuchen. Möglich ist auch, dass keine austrittseitige Öffnung Ö.a vorhanden ist und die Messkammer-Probe durch die eintrittsseitige Öffnung Ö.e hindurch wieder aus der Messkammer 3 herausfließt.

Der elektrochemische Sensor 12 umfasst
- eine Messelektrode 20, die durch einen Kontaktierungsdraht 34 elektrisch kontaktiert ist,
- eine Gegenelektrode 21, die durch einen Kontaktierungsdraht 33 elektrisch kontaktiert ist,
- einen Elektrolyten 28 zwischen den beiden Elektroden 20 und 21,
- einen Verbindungsdraht 12, der die beiden Kontaktierungsdrähte 33 und 34 elektrisch miteinander verbindet und in dem ein elektrischer Messwiderstand 29 angeordnet ist, und
- einen Stromstärken-Sensor 38, der die Stärke I des durch den Verbindungsdraht 12 fließenden Stroms misst.

Ein solcher elektrochemischer Sensor 12 wird nachfolgend auch als Membran-Elektroden-Elektrolyt-Einheit (MPEE) bezeichnet.

Der Elektrolyt 28 ist oder umfasst ein elektrisch leitendes Medium, beispielsweise mit Wasser verdünnte Schwefelsäure oder Phosphorsäure oder Perchlorsäure. In dem Elektrolyten 28 können sich Ionen bewegen. Bevorzugt stellt eine poröse Membrane den Elektrolyten 28 bereit. Der Elektrolyt 28 stellt eine ionisch leitfähige Verbindung zwischen der Messelektrode 20 und der Gegenelektrode 21 her, isoliert aber die beiden Elektroden 20 und 21 elektrisch voneinander.

Der Sensor 12 ist so ausgestaltet, dass die Messkammer-Probe Pr nur die Messelektrode 20 erreicht, aber nicht die Gegenelektrode 21. Im gezeigten Beispiel befindet die Messelektrode 20 sich an einer Wand der Messkammer 3, und die Wandung 40 und der Elektrolyt 28 verhindern, dass eine relevante Menge der Messkammer-Probe Pr die Gegenelektrode 21 erreicht.

Die beiden Kontaktierungsdrähte 33 und 34 sind elektrisch leitend und aus einem Material, welches vom Elektrolyten 28 nicht chemisch angegriffen wird, beispielsweise aus Platin oder Gold. Auch die Elektroden 20 und 21 sind aus einem chemisch resistenten Material, beispielsweise ebenfalls aus Platin oder Gold. In vielen Fällen fungiert das chemisch resistente Material der Elektroden 20, 21 zusätzlich als Katalysator für eine chemische Reaktion, die von der zu detektierenden Substanz abhängt und zur Messung verwendet wird.

In einer Realisierungsform funktioniert der elektrochemische Sensor 12 nach dem Prinzip der Brennstoffzelle. Die chemische Reaktion, die zur Messung verwendet wird, umfasst den Schritt, dass in der Messkammer 3 der Atemalkohol in der Messkammer-Probe Pr oxidiert wird. Idealerweise wird die gesamte Menge des Atemalkohols in der Messkammer-Probe Pr oxidiert.

Als Folge der chemischen Reaktion fließt ein elektrischer Strom zwischen der Messelektrode 20 und der Gegenelektrode 21 und damit durch den Verbindungsdraht 12. Der Stromstärken-Sensor 38 misst ein Maß für die elektrische Ladung, also für die gesamte Menge des elektrischen Stroms, der durch den Verbindungsdraht 12 fließt (Prinzip der Coulometrie). In der Regel fließt elektrischer Strom so lange, bis alles brennbare Gas, hier also Atemalkohol, in der Messkammer 3 oxidiert ist. Bei einem gegebenen Volumen der Messkammer-Probe Pr in der Messkammer 3 ist die gemessene elektrische Ladung umso höher, je mehr Atemalkohol die Messkammer-Probe Pr vor dem Oxidieren enthält. Die gemessene elektrische Ladung ist daher ein Maß für den Atemalkohol-Gehalt in der Messkammer-Probe Pr und somit für den Gehalt an Alkohol im Blut des Probanden.

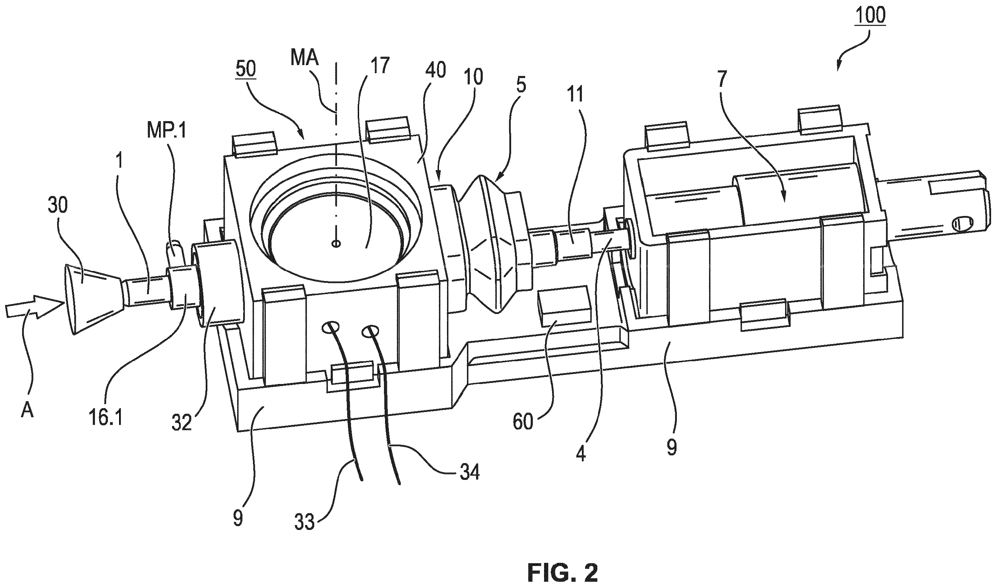

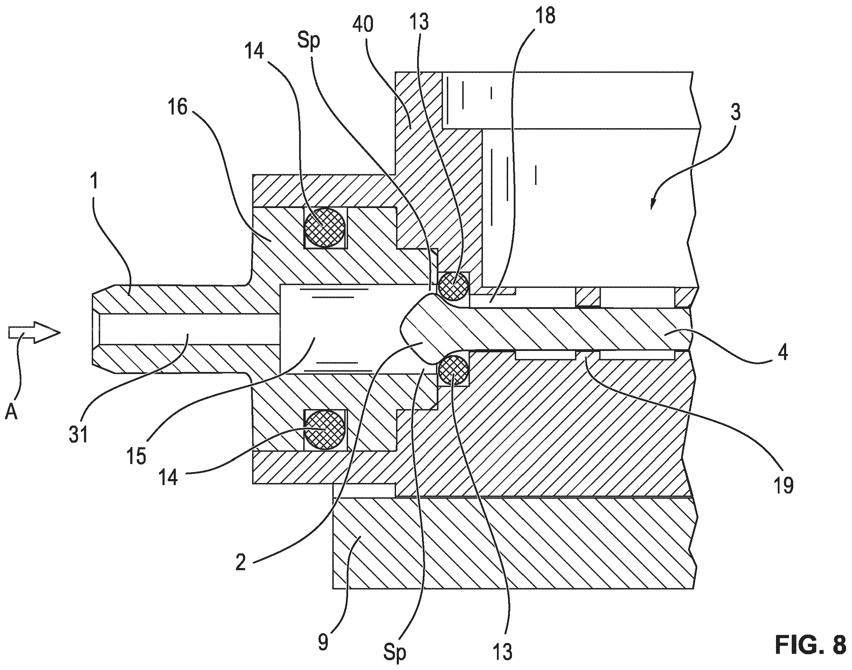

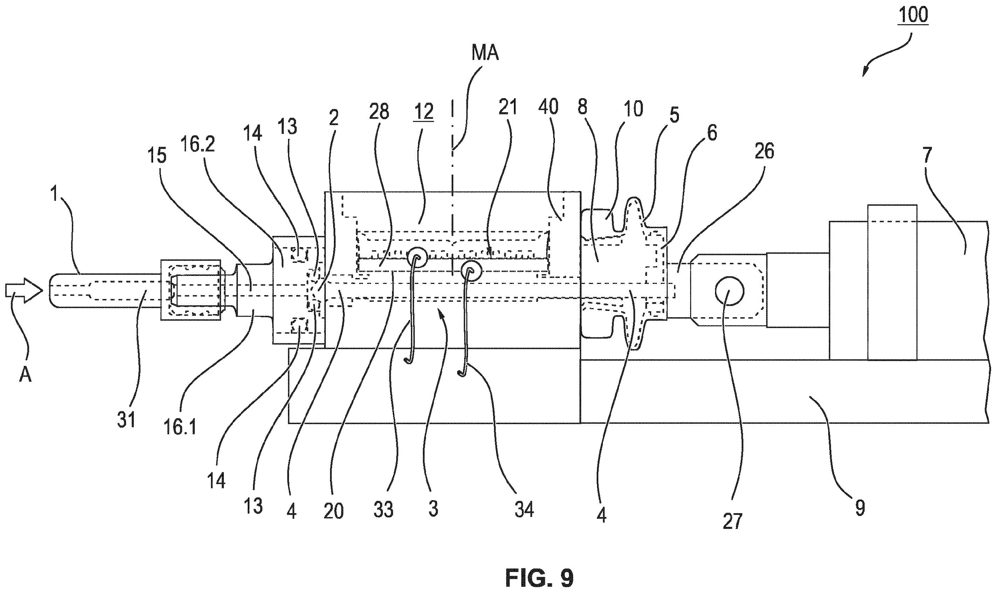

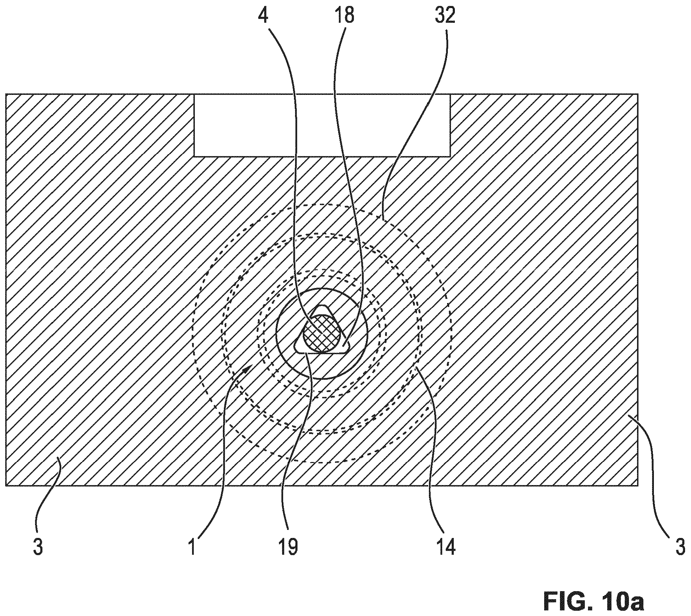

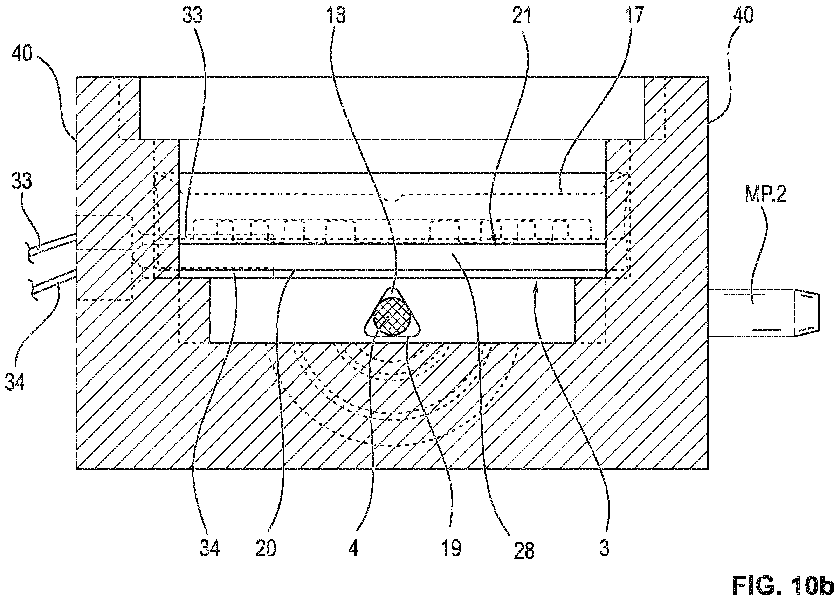

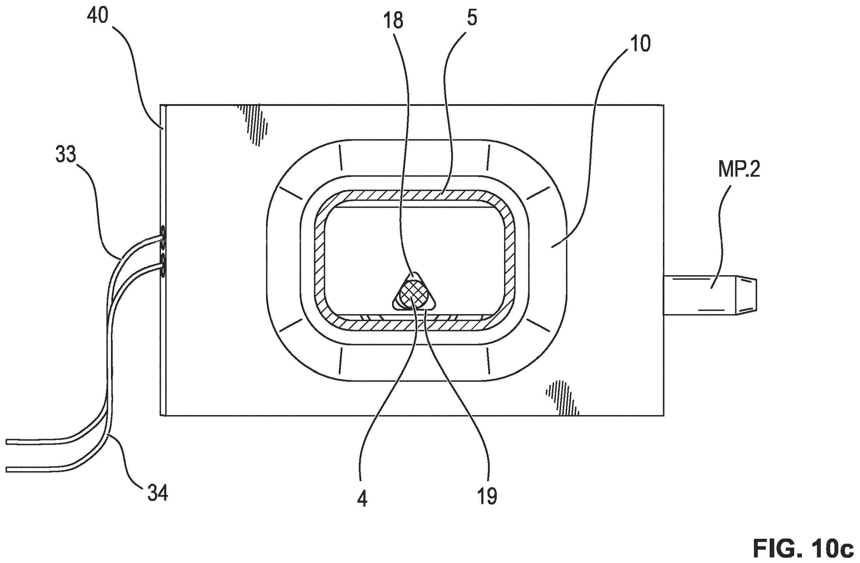

Figur 2 bis Figur 7 zeigen eine erste Ausgestaltung des erfindungsgemäßen Analysegeräts 100. Figur 9 zeigt eine zweite Ausgestaltung. Figur 8 und Figur 10 sind für beide Ausgestaltungen gültig.

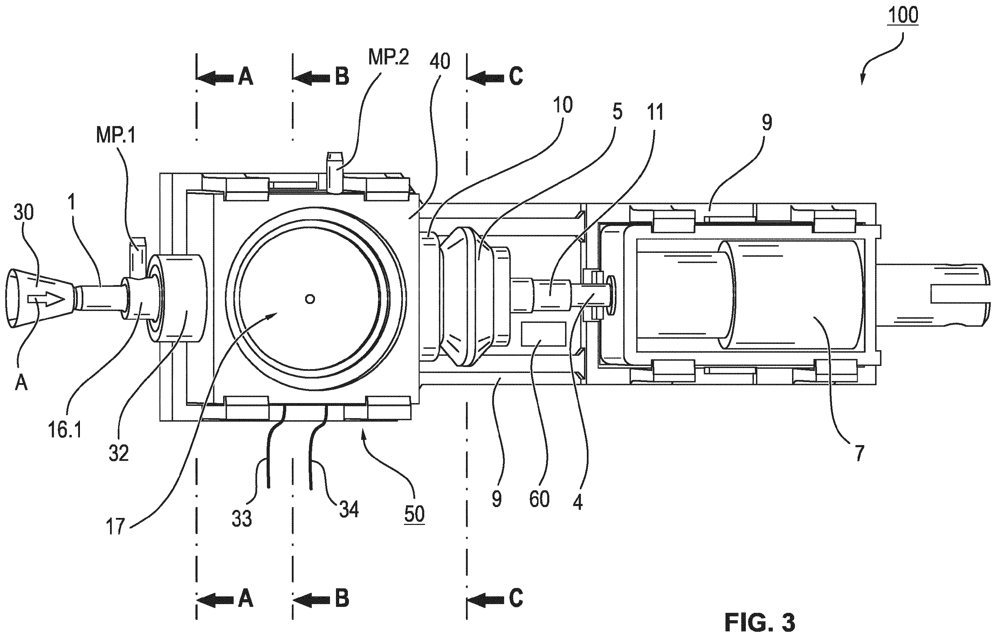

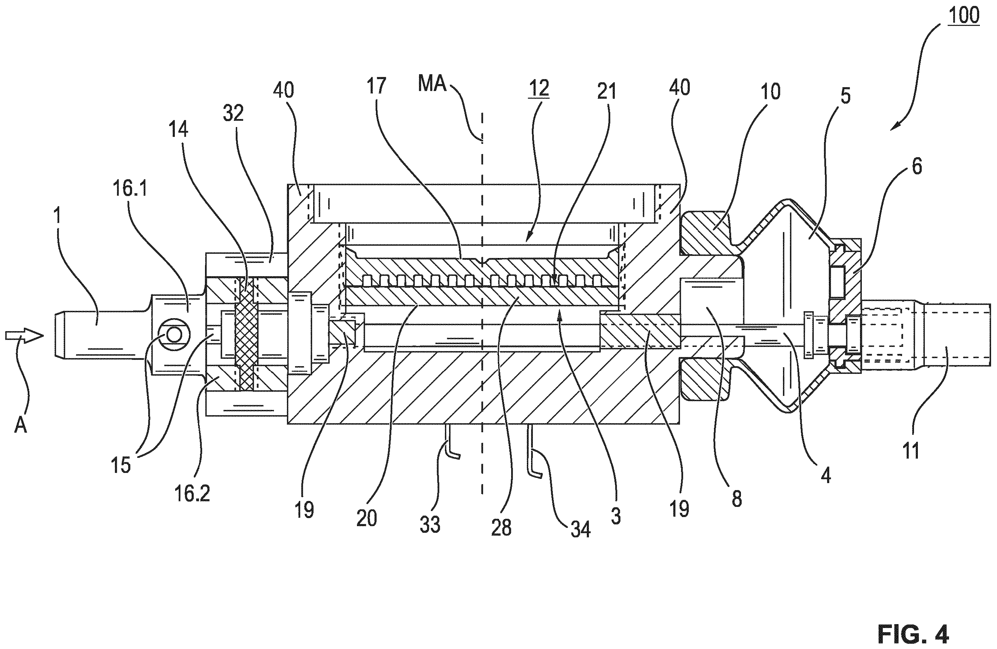

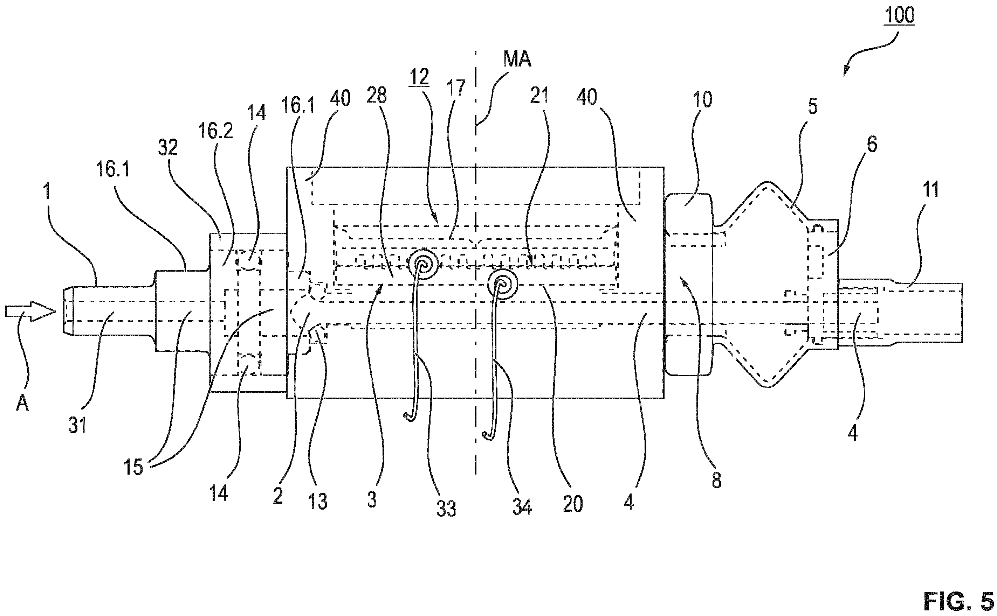

Figur 2 und Figur 3 zeigen das erfindungsgemäße Analysegerät 100 gemäß der ersten Ausgestaltung in einer perspektivischen Darstellung, Figur 4 und Figur 5 in zwei Querschnittsdarstellungen.

Auf einem Gestell 9 des Analysegeräts 100 sind folgende weitere Bestandteile montiert:
- ein nur schematisch gezeigtes Mundstück 30,
- ein Probeneinlass 1,
- ein Verbindungsstück 16, welches aus einem kleineren Teil 16.1 und einem größeren Teil 16.2 besteht, wobei die beiden Teile 16.1, 16.2 fest miteinander verbunden sind,
- eine Sensor-Anordnung 50 mit einer Messkammer 3 und einem elektrochemischen Sensor 12, wobei die Sensor-Anordnung 50 z.B. so wie in Figur 1 gezeigt aufgebaut sein kann,
- ein einströmseitiges Anschlussstück 32, welches flussaufwärts von der Messkammer 3 an einer Wandung 40 der Messkammer 3 befestigt ist und das Teil 16.2 umgibt,
- ein ausströmseitiges Anschlussstück 10, welches flussabwärts von der Messkammer 3 an der Wandung 40 der Messkammer 3 befestigt ist,
- eine linear verschiebliche Stange 4,
- eine Verbindungs-Hülse 11, durch die hindurch die Stange 4 geführt ist,
- ein Balg 5, der als die Ansaug-Kammereinheit des Ausführungsbeispiels fungiert,
- eine Platte 6 im Balg 5, wobei die Platte 6 als das Kammer-Veränderungselement fungiert,
- ein optionaler erster Messpunkt MP.1 für eine nachfolgend beschriebene Druckmessung oder Volumenfluss-Messung,
- ein optionaler zweiter Messpunkt MP.2 für eine andere Druckmessung oder für diese Volumenfluss-Messung,
- ein Stellantrieb, der einen Gegenstand in zwei entgegengesetzte Richtungen bewegen kann und dessen Funktion weiter unten beschrieben wird,
- ein schematisch gezeigtes signalverarbeitende Steuergerät (control unit) 60, welches jeweils ein Signal von der Sensor-Anordnung 50 und von einem Volumenfluss-Sensor oder einem Druck-Sensor empfängt und den Stellantrieb anzusteuern vermag und
- eine nicht gezeigte Spannungsversorgungseinheit, beispielsweise mindestens ein Akkumulator, welche den Stellantrieb mit elektrischer Energie versorgt.

Die Bezeichnungen "vorne" und "hinten" sowie "flussaufwärts" und "flussabwärts" beziehen sich auf die Fließrichtung eines Gases vom Probeneinlass 1 zum Balg 5, in Figur 2 bis Figur 9 also von links nach rechts.

Das Mundstück 30 lässt sich auf den Probeneinlass 1 aufsetzen und wieder vom Probeneinlass 1 abziehen. In einer Ausgestaltung umgibt das aufgesetzte Mundstück 30 den Probeneinlass 1. Das Mundstück 30 hat die Form eines Trichters, wobei dieser Trichter sich zum Probeneinlass 1 hin verjüngt, wenn das Mundstück 30 aufgesetzt ist. Dank dieser Trichterform entsteht im Inneren des Mundstücks 30 ein Überdruck, wenn ein Proband eine Atemprobe A eingibt.

Das Mundstück 30 gehört zur Eingabeeinheit des Ausführungsbeispiels, der Probeneinlass 1 und das Verbindungsstück 16 zur Eingabe-Fluidführungseinheit. Eine nachfolgend beschriebene Eingabe-Fluidverbindung ist durch die Eingabe-Fluidführungseinheit hindurch geführt und vermag das Mundstück 30 mit der Messkammer 3 zu verbinden.

In das Mundstück 30 ist eine Öffnung eingelassen, durch welche hindurch die eingegebene Atemprobe zum Probeneinlass 1 fließen kann. Bevorzugt sind in das Mundstück 30 weitere Öffnungen (nicht gezeigt) eingelassen. Durch diese weiteren Öffnungen kann Atemluft in die Umgebung entweichen, insbesondere dann, wenn im Mundstück 30 ein Überdruck entstanden ist. Dadurch wird die Gefahr reduziert, dass bei einem Überdruck im Mundstück 30 ein Teil der Atemprobe A zurück zum Probanden gelangt. Ein Mundstück mit solchen Öffnungen wird beispielhaft in DE 10 2017 008 008 A1 beschrieben.

Die Messkammer 3 ist von der Wandung 40 und einer Deckplatte 17 umgeben. Der Sensor 12 ist unter der Deckplatte 17 angeordnet. Im gezeigten Ausführungsbeispiel hat die Wandung 40 der Sensor-Anordnung 50 eine Außenkontur in Form eines Quaders und eine Innenkontur in Form eines Zylinders. Andere geometrische Formen sind ebenfalls möglich. Der Sensor 12 und die Messkammer 3 sind rotationssymmetrisch zu derselben Mittelachse MA. Diese Mittelachse MA steht senkrecht auf der Zeichenebene von Figur 3 und liegt in den Zeichenebenen von Figur 4 und Figur 5.

Im gezeigten Beispiel umfasst der Stellantrieb einen Hubmagneten 7 und eine Rückstelleinheit in Form einer Feder, die sich am Gestell 9 abstützt und mit dem Hubmagneten 7 verbunden ist. Die nicht gezeigte Spannungsversorgungseinheit ist mit dem Hubmagneten 7 elektrisch verbunden. Auch andere Ausgestaltungen eines Stellantriebs sind möglich, beispielsweise ein Elektromotor oder eine Kolben-Zylinder-Einheit. Denkbar ist sogar ein manueller Antrieb.

Außerdem werden in den Querschnittsdarstellungen von Figur 4 und Figur 5 folgende Bestandteile des Analysegeräts 100 gezeigt:
- ein Ventil mit einem Verschlussteil und einem Verschlussteil-Sitz 13,
- ein Hohlraum 31 in Form einer Röhre im Probeneinlass 1,
- ein Hohlraum 15 in Form einer Röhre im Inneren des Verbindungsstücks 16,
- die Platte 6 im Balg 5, wobei die Platte 6 fest mit der Stange 4 verbunden ist,
- eine Führungseinheit 19, welche die Stange 4 linear entlang der Längsachse der Stange 4 führt und eine seitliche Bewegung oder Drehung oder Verkantung der Stange 4 verhindert,
- einen Schnitt durch den Sensor 12 mit der Messelektrode 20, der Gegenelektrode 21 und dem Elektrolyten 28 und
- ein Schnitt durch die Wandung 40 der Messkammer 3.

Außerdem wird in Figur 4 und Figur 5 gezeigt, wie die linear verschiebliche Stange 4 und die Verbindungs-Hülse 11 den Dichtkegel 2 mit dem Hubmagneten 7 verbinden.

Im Ausführungsbeispiel hat das Verschlussteil 2 die Form eines Dichtkegels, und der Verschlussteil-Sitz 13 hat die Form eines Dichtrings, der bevorzugt elastisch ist. Der Durchmesser des Dichtkegels 2 ist bevorzugt größer als der Durchmesser der Stange 4. Dadurch ist es möglich, den Durchmesser des Dichtkegels 2 so groß wie möglich und den Durchmesser der Stange 4 so klein wie möglich auszugestalten. In jeder Position der Stange 4 befindet der Dichtkegel 2 sich im Hohlraum 15. Zwischen dem Dichtkegel 2 und der Innenwand des Hohlraums 15 tritt ein umlaufender Spalt Sp auf, vgl. Figur 5 und Figur 8.

Der Verschlusselement-Sitz (Dichtring) 13 umgibt dasjenige Ende der Stange 4, das an den Dichtkegel 2 angrenzt, und ist in eine Aussparung in der Wandung 40 eingelassen. Die Stange 4 ist durch die Messkammer 3 hindurchgeführt, vgl. Figur 4 und Figur 5. Im gezeigten Ausführungsbeispiel steht die Längsachse der Stange 4 senkrecht auf der Mittelachse MA der zylinderförmigen Messkammer 3 und liegt in den Zeichenebenen von Figur 3, Figur 4 und Figur 5. **In** einer Ausgestaltung ist auf der Stange 4 mindestens ein optionales Mischelement (nicht gezeigt) in Form eines flächigen Bauteils fest montiert. Das oder jedes Mischelement befindet sich in jeder Position der Stange 4 im Inneren der Messkammer 3.

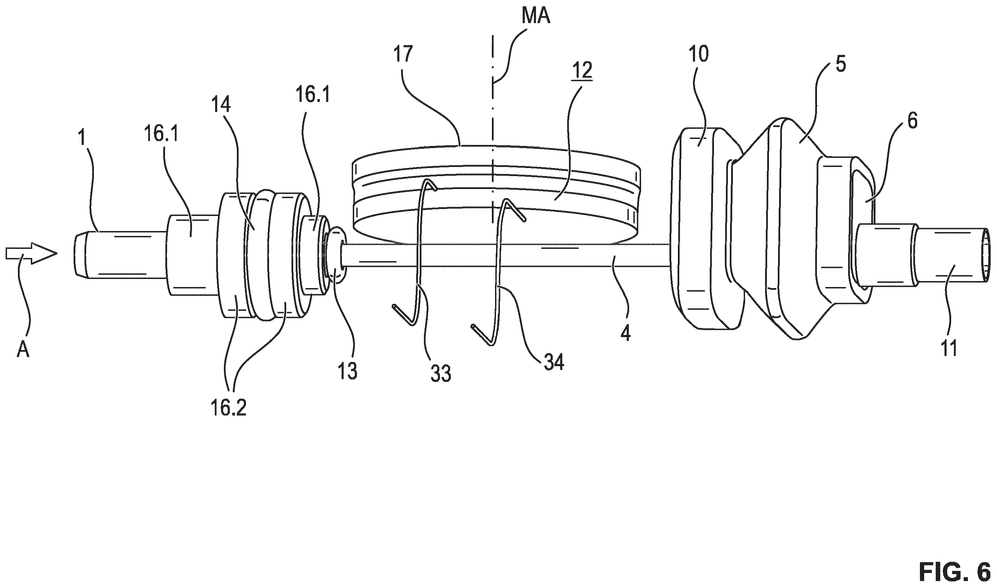

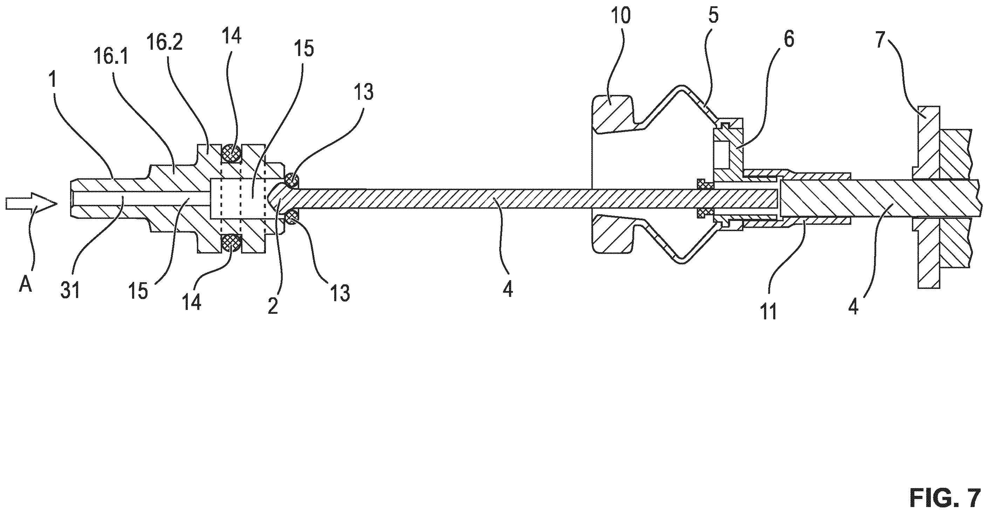

Figur 6 und Figur 7 zeigen in einer perspektivischen Darstellung bzw. in einer Querschnittsdarstellung den Probeneinlass 1, das Ventil 2, 13 sowie die Eingabe-Fluidverbindung zwischen dem Probeneinlass 1 und der Messkammer 3. Figur 8 veranschaulicht in einer schematischen Querschnittsdarstellung und in einer von Figur 2 bis Figur 7 leicht abweichenden Realisierungsform die Eingabe-Fluidverbindung zwischen dem Probeneinlass 1 und der Messkammer 3.

In den gezeigten Ausgestaltungen steht die Messkammer 3 ausschließlich über die Eingabe-Fluidverbindung mit dem Mundstück 30 in einer Fluidverbindung, und dies nur bei vollständig oder wenigstens teilweise geöffnetem Ventil 2, 13.

Möglich sind auch zwei alternative Ausgestaltungen, die beide nicht gezeigt werden:
- Bei der ersten alternativen Ausgestaltung steht das Mundstück 30 über eine separate Ausgabe-Fluidverbindung in einer Fluidverbindung mit der Umgebung. Bevorzugt zweigt diese Ausgabe-Fluidverbindung flussaufwärts vom Ventil 2, 13 von der Eingabe-Fluidverbindung ab. Bevorzugt ist diese Ausgabe-Fluidverbindung geschlossen, wenn das Ventil 2, 13 geöffnet ist, und ist geöffnet, wenn das Ventil 2, 13 geschlossen ist. Bei geöffneter Ausgabe-Fluidverbindung fließt Atemluft, die in das Mundstück 30 eingegeben wurde, durch die Ausgabe-Fluidverbindung hindurch in die Umgebung, insbesondere bei geschlossenem Ventil 2, 13. Diese Ausgestaltung reduziert die Gefahr, dass Gas, das der Proband in das Mundstück 30 eingegeben hat, zurück zum Probanden fließt.
- Bei der zweiten Ausgestaltung steht die Messkammer 3 über eine Auslass-Fluidverbindung in einer Fluidverbindung mit der Umgebung. Die Messkammer 3 lässt sich über diese Auslass-Fluidverbindung hindurch ausspülen. Bevorzugt ist in dieser Auslass-Fluidverbindung ein Ventil angeordnet, welches nur dann geöffnet ist, wenn Gas aus der Messkammer 3 entfernt werden soll. Diese Ausgestaltung vermeidet, dass beim Spülen der Messkammer 3 Gas aus der Messkammer 3 in das Mundstück 30 geleitet oder gefördert wird.

In den Darstellungen von Figur 2 bis Figur 7 wird der Balg 5 in einem Zustand mit maximalem Volumen gezeigt. Die Stange 4 koppelt diesen Zustand in der ersten Ausgestaltung gemäß Figur 2 bis Figur 7 mit einem Zustand, in dem das Ventil 2, 13 in der verschließenden Endposition ist.

Figur 9 zeigt den Balg 5 in einem Zustand mit minimalem Volumen. **In** der zweiten Ausgestaltung gemäß Figur 9 koppelt die Stange 4 diesen Zustand mit dem Zustand, in dem das Ventil 2, 13 in der verschließenden Endposition ist. Gleiche Bezugszeichen haben gleiche Bedeutungen wie in Figur 2 bis Figur 7.

In Figur 9 werden außerdem folgende Bestandteile gezeigt:
- ein Verbindungselement 26 zwischen der Platte 6 und dem Hubmagneten 7 und
- ein Bolzen, der durch eine Aussparung im Verbindungselement 26 und durch eine Aussparung in einer Stange des Hubmagneten 7 hindurchgeführt ist.

Der Sensor 12 kann sich dank des Bolzens 27 relativ zum Hubmagneten 7 um die Längsachse des Bolzens 27 drehen.

Figur 10 zeigt drei Schnitte durch das Analysegerät 100, wobei die Achse der Stange 4 in jedem Schnitt senkrecht auf der jeweiligen Zeichenebene steht und die Betrachtungsrichtung von Hubmagneten 7 zum Probeneinlass 1 gerichtet ist. Diese drei Schnitte gelten für beide Ausgestaltungen des Analysegeräts 100. Figur 10 a) zeigt einen Schnitt in der Ebene A - A von Figur 3, Figur 10 b) einen Schnitt in der Ebene B - B und Figur 10 c) einen Schnitt in der Ebene C - C.

Folgende weiteren Bestandteile des Analysegeräts 100 werden in Figur 6, Figur 7 und / oder Figur 8 gezeigt:
- eine röhrenförmige Aussparung 18, welche die Stange 4 umgibt und eine annähernd dreieckige Querschnittsfläche aufweist,
- eine Führungseinheit 19 für die Stange 4 und
- ein weiterer Dichtring 14 um die Röhre 16.

Die Hohlräume 31 und 15 bilden zusammen eine Röhre, die sich in der Aussparung 18 fortsetzt. Im Ausführungsbeispiel stellen die Röhre 31, 15, der Spalt Sp und die Aussparung 18 zusammen die Eingabe-Fluidverbindung zwischen dem Probeneinlass 1 und der Messkammer 3 her. Der Dichtkegel 2 ist zwischen einer verschließenden Endposition, in welcher die Eingabe-Fluidverbindung 31, 15, Sp, 18 unterbrochen ist, und einer freigebenden Endposition, in welcher die Eingabe-Fluidverbindung 31, 15, Sp, 18 freigegeben ist, hin und her beweglich. In der verschließenden Endposition, die in den Figuren gezeigt ist, liegt der Dichtkegel 2 fluiddicht am Verschlusselement-Sitz (Dichtring) 13 an. Indem der Dichtkegel 2 vom Verschlusselement-Sitz 13 weg und auf den Probeneinlass 1 zu (erste Ausgestaltung) bzw. von dem Probeneinlass 1 weg (zweite Ausgestaltung) bewegt wird, wird der Dichtkegel 2 linear in die freigebende Endposition bewegt.

Wenn der Dichtkegel 2 in der freigebenden Endposition oder in einer Zwischenposition zwischen der freigebenden und der verschließenden Endposition ist, so ist eine Eingabe-Fluidverbindung 31, 15, Sp, 18 zwischen dem Mundstück 30 und der Messkammer 3 hergestellt. Diese Eingabe-Fluidverbindung 31, 15, Sp, 18 führt durch folgende Bestandteile hindurch:
- die Röhre 31,
- den Hohlraum 15,
- den Spalt Sp zwischen dem Dichtkegel 2 und der Innenwand des Hohlraums 15,
- den vom Dichtring 13 umschlossenen Innenraum und
- die Aussparung 18 um die Stange 4 herum.

Wenn der Dichtkegel 2 in der verschließenden Endposition ist, also am Dichtring 13 anliegt, so ist diese Eingabe-Fluidverbindung 31, 15, Sp, 18 unterbrochen.

Die Stange 4 und die Verbindungs-Hülse 11 verbinden den Dichtkegel 2 mit dem Hubmagneten 7. Der Stellantrieb mit dem Hubmagneten 7 und der nicht gezeigten Feder vermag die Stange 4 in beide Richtungen linear zu bewegen und dadurch den Dichtkegel 2 zwischen der verschließenden Endposition und der freigebenden Endposition hin und her zu bewegen. Die Stange 4 ist durch die Verbindungs-Hülse 11 hindurch geführt.

Die Stange 4, die Verbindungs-Hülse 11 und die Platte 6 sind dergestalt mechanisch miteinander verbunden, dass sie sich nicht relativ zueinander bewegen können. Die Verbindungs-Hülse 11 überträgt eine Bewegung der Stange 4 auf die Platte 6. Zusammen mit der Stange 4 vermag der Hubmagnet 7 auch die Verbindungs-Hülse 11 und damit die Platte 6 linear zu bewegen.

Der Balg 5 ist an der Seite, die dem Probeneinlass 1 zugewandt ist, mechanisch mit der Wandung 40 verbunden. Das Anschlussstück 10 umgibt den Balg 5. Die Platte 6 begrenzt an der gegenüberliegenden Seite den Balg 5. Eine lineare Bewegung der Platte 6 auf den Probeneinlass 1 zu drückt den Balg 5 zusammen und überführt den Balg 5 in den Zustand mit minimalem Volumen. Eine lineare Bewegung der Platte 6 in die entgegengesetzte Richtung zieht den Balg 5 auseinander und überführt den Balg 5 in den Zustand mit maximalem Volumen. Der Balg 5 steht in einer Ansaug-Fluidverbindung 8 mit der Messkammer 3.

Der Hubmagnet 7, die Feder, der Balg 5 und die Platte 6 bilden zusammen eine Verdränger-Pumpe. Anstelle eines Hubmagneten 7 kann das Analysegerät 100 auch einen sonstigen ansteuerbaren Stellantrieb aufweisen, wobei dieser Stellantrieb die Stange 4 in beide Richtungen bewegen und in einer Endposition halten kann. Denkbar ist auch ein manueller Antrieb.

Anstelle eines Balgs 5 und einer Platte 6 lässt sich auch eine Kolben-Zylinder-Einheit (nicht gezeigt) verwenden, wobei der Stellantrieb 7 den Kolben relativ zum Zylinder zu bewegen vermag. Möglich ist auch, dass der gerade erwähnte sonstige Stellantrieb den Kolben einer Kolben-Zylinder-Einheit zu bewegen vermag. Möglich ist auch, dass ein Elektromotor die Stange 4 in beide Richtungen linear zu bewegen vermag.

Der optionale erste Messpunkt MP.1 steht in einer Fluidverbindung mit dem Probeneinlass 1 oder mit dem Hohlraum 15 und damit in einer Fluidverbindung mit der gerade beschriebenen Eingabe-Fluidverbindung 31, 15, Sp, 18, welche das Mundstück 30 mit der Messkammer 3 verbindet. Daher steht der erste Messpunkt MP.1 auch dann in einer Fluidverbindung mit dem Mundstück 30, wenn das Ventil 2, 13 geschlossen ist. Möglich ist auch, dass der erste Messpunkt MP.1 in einer Fluidverbindung mit der Aussparung 18 steht. Der optionale zweite Messpunkt MP.2 ist zwischen der Messkammer 3 und dem Hubmagneten 7 angeordnet und steht in einer Fluidverbindung mit der Messkammer 3.

Zwei nicht gezeigte Drucksensoren messen den Druck am ersten Messpunkt MP.1 bzw. am zweiten Messpunkt MP.2. An jedem Abtast-Zeitpunkt einer Folge von Abtast-Zeitpunkten werden jeweils zwei Druckmessungen durchgeführt. Bevorzugt messen diese beiden Drucksensoren die jeweilige Druckdifferenz zum Umgebungsdruck in der Umgebung des Analysegeräts 100.

In einer Ausgestaltung werden die Messwerte desjenigen Drucksensors, der mit dem ersten Messpunkt MP.1 verbunden ist, verwendet, um näherungsweise den Volumenfluss in das Mundstück 30 und damit das Volumen derjenigen Menge der Atemprobe A zu ermitteln, die bislang in das Mundstück 30 eingegeben wurde. Zumindest bei geschlossenem Ventil 2, 13 bewirkt diese eingegebene Menge der Atemprobe A im Wesentlichen, dass der Druck im Inneren des trichterförmigen Mundstücks 30 vergrößert wird. Die Schlitze im Mundstück 30 können diesen Überdruck nur teilweise abbauen. Die Information, welches Volumen die bislang eingegebene Menge hat, lässt sich für das Auslösen des Vorgangs verwenden, das Ventil 2, 13 zu öffnen. Wie bereits dargelegt, soll nur Luft aus der Lunge des Probanden in die Messkammer 3 gelangen, aber nicht Luft aus seinem Mund und seinen oberen Atemwegen. Wie diese gewünschte Wirkung erzielt wird, wird weiter unten näher beschrieben

In einer Ausgestaltung werden die Messwerte des Drucksensors, der mit dem zweiten Messpunkt MP.2 verbunden ist, verwendet, um den zeitlichen Verlauf des Drucks in der Messkammer 3 zu messen. Aus diesem zeitlichen Verlauf des Drucks sowie dem Volumen der Messkammer 3, welches durch die Konstruktion des Analysegerät 100 bekannt ist, lässt sich ein Schätzwert für die Menge der Messkammer-Probe Pr ableiten.

In einer weiteren Ausgestaltung, die sich mit den gerade beschriebenen beiden Ausgestaltungen kombinieren lässt, leitet ein nicht gezeigter Volumenfluss-Sensor die Differenz zwischen dem gemessenen Druck am ersten Messpunkt MP.1 und dem gemessenen Druck am zweiten Messpunkt MP.2 ab. Diese Druckdifferenz ist ein Maß für den aktuellen Volumenfluss aus der und in die Messkammer 3. Optional wird außerdem automatisch überprüft, ob das Ventil 2, 13 dicht ist, also in der verschließenden Position tatsächlich die Eingabe-Fluidverbindung unterbricht.

Figur 8 veranschaulicht, wie die Stange 4 (in Figur 8 gestrichelt gezeigt) geführt wird. Im gezeigten Beispiel hat die Aussparung 18 einen dreieckigen Querschnitt, sodass eine Atemprobe A an der Stange 4 vorbei zur Messkammer 3 fließen kann. Außerdem wird die Stange 4 von der Führungseinheit 19 linear geführt. Dank dieser Führung kann die Stange 4 sich nur linear in zwei Richtungen parallel zu ihrer eigenen Längsachse bewegen, aber nicht seitlich bewegen oder verkanten.

Figur 10 zeigt die dreieckige Querschnittsfläche des Hohlraums 18.

Im Folgenden wird beschrieben, wie das Analysegerät 100 eine Atemprobe A aufnimmt und untersucht.

Vor einem Einsatz befindet das Analysegerät 100 sich in einem Ruhezustand. Auf den Probeneinlass 1 ist kein Mundstück 30 aufgesetzt. Eine mechanische oder pneumatische Feder (nicht gezeigt) des Stellantriebs stützt sich am Gestell 9 ab und hält die Stange 4 bei der ersten Ausgestaltung in einer Position, in der die Stange 4 den maximal möglichen Abstand vom Probeneinlass 1 hat, und bei der zweiten Ausgestaltung in einer Position mit minimal möglichem Abstand vom Probeneinlass 1. Der Hubmagnet 7 ist deaktiviert, d.h. nicht von Strom durchflossen. Dank der Feder zieht die Platte 6 den Balg 5 in der ersten Ausgestaltung gemäß Figur 2 bis Figur 7 auseinander, sodass der Balg 5 das maximale Volumen hat. Bei der zweiten Ausgestaltung drückt die Platte 6 im Ruhezustand dank der Feder den Balg 5 zusammen, sodass der Balg 5 das minimale Volumen hat.

Der Dichtkegel 2 ist vor einem Einsatz in der verschließenden Endposition, und das Ventil 2, 13 verschließt die Eingabe-Fluidverbindung 15, 18 zwischen dem Probeneinlass 1 und der Messkammer 3. Daher steht die Messkammer 3 nicht in einer Fluidverbindung mit der Umgebung. Partikel, Substanzen und sonstige Umgebungseinflüsse können daher nicht auf den elektrochemischen Sensor 12 einwirken, solange das Analysegerät 100 im Ruhestand ist, und umgekehrt ist die Gefahr gering, dass Bestandteile des Elektrolyten 28 den elektrochemischen Sensor 12 oder gar die Messkammer 3 verlassen, beispielsweise aufgrund von Verdunstung.

In einer Ausgestaltung wird das Mundstück 30 für die Eingabe einer einzigen Atemprobe A verwendet und danach entsorgt. In einer anderen Ausgestaltung wird das Mundstück 30 nach der Eingabe einer Atemprobe A desinfiziert und anschließend erneut verwendet.

In beiden Ausgestaltungen wird das Mundstück 30 erst dann mit dem Rest des Analysegeräts 100 verbunden, wenn ein Einsatz das Analysegeräts 100 beginnt und ein Proband eine Atemprobe A eingibt. Bevorzugt löst das Ereignis, dass das Mundstück 30 auf den Probeneinlass 1 aufgesetzt wird, den Schritt aus, das Analysegerät 100 aus dem Ruhezustand in einen Einsatzzustand zu überführen. Beispielsweise detektiert ein Kontaktschalter das Ereignis, dass das Mundstück 30 auf den Probeneinlass 1 aufgesetzt worden ist.

Bei einem Einsatz gibt ein Proband eine Atemprobe A in das aufgesetzte Mundstück 30 ein. Diese Atemprobe A enthält anfangs ausgeatmete Luft aus dem Mund und den oberen Atemwegen und anschließend ausgeatmete Luft aus der Lunge des Probanden. Das Analysegerät 100 untersucht idealerweise ausschließlich ausgeatmete Luft aus der Lunge. Daher bleibt das Ventil 2, 13 zunächst geschlossen, auch wenn der Proband bereits damit begonnen hat, eine Atemprobe A in das Mundstück 30 einzugeben. Wie bereits erwähnt, umfasst das Mundstück 30 bevorzugt mehrere weitere Öffnungen, sodass die eingegebene Atemprobe A vollständig in die Umgebung austreten kann, solange das Ventil 2, 13 geschlossen ist, und nicht dem Probanden ins Gesicht geblasen wird.

Das Analysegerät 100 detektiert im Einsatzzustand automatisch das Auftreten eines vorgegebenen öffnenden Ereignisses.

Bevor das öffnende Ereignis eingetreten ist, ist das Ventil 2, 13 geschlossen, und die eingegebene Luft entweicht durch die Schlitze oder durch die Ausgabe-Fluidverbindung hindurch wieder aus dem Mundstück 30. Dadurch wird sichergestellt, dass tatsächlich Luft aus der Lunge des Probanden in die Messkammer 3 fließt und insbesondere keine nennenswerte Menge von Luft aus dem Mund und den oberen Atemwegen.

Dieses öffnende Ereignis ist beispielsweise dann eingetreten, wenn seit dem Schritt, das Mundstück 30 aufzusetzen, eine vorgegebene Zeitspanne verstrichen ist. Oder das öffnende Ereignis ist eingetreten, wenn seit dem Schritt, das Mundstück 30 aufzusetzen, eine vorgegebene Menge der Atemprobe A in das Mundstück 30 eingegeben wurde oder der Proband den Schritt, eine Atemprobe A einzugeben, beendet hat. Bei der zweiten Alternative misst ein Sensor ein Maß für das Volumen von Gas, das in das Mundstück 30 eingegeben wurde, oder für den Volumenfluss von Gas in das Mundstück 30. Dies wird weiter unten näher beschrieben.

Die Detektion, dass das öffnende Ereignis eingetreten ist, löst in der ersten Ausgestaltung gemäß Figur 2 bis Figur 7 folgende Schritte aus:
- Ein Stromkreis wird geschlossen, und elektrischer Strom aktiviert den Hubmagneten 7.
- Der aktivierte Hubmagnet 7 schiebt die Stange 4 gegen die Kraft der Feder auf den Probeneinlass 1 zu.
- Das Verschieben der Stange 4 auf den Probeneinlass 1 zu bewirkt, dass der Dichtkegel 2 vom Dichtring (Verschlusselement-Sitz) 13 weggeschoben und zu dem Probeneinlass 1 hin geschoben wird. Dadurch wird das Ventil 2, 13 geöffnet, nämlich in die freigebende Endposition bewegt. Dadurch wird die oben beschriebene Eingabe-Fluidverbindung zwischen der Eingabeeinheit (dem Mundstück 30 und dem Probeneinlass 1) und der Messkammer 3 freigegeben.
- Das Verschieben der Stange 4 bewirkt außerdem, dass die Platte 6 den Balg 5 zusammendrückt.
- Weil der Balg 5 zusammengedrückt wird, fließt Gas aus dem Balg 5 durch die Ansaug-Fluidverbindung 8 hindurch in die Messkammer 3. Dadurch wird vorhandenes Gas aus der Messkammer 3 durch die Eingabe-Fluidverbindung 18, 15 hindurch und durch den Probeneinlass 1 hindurch aus dem Analysegerät 100 herausgeschoben. Dadurch wird die Messkammer 3 gespült. Das herausgespülte Gas in der Messkammer 3 kann von einer vorhergehenden Eingabe stammen.
- Der Druck, den das Zusammendrücken des Balgs 5 verursacht, ist wesentlich größer als der Druck, den die Eingabe der Atemprobe A in das Mundstück 30 bewirkt. Daher fließt keine nennenswerte Menge der eingegebenen Atemprobe A in die Eingabe-Fluidverbindung hinein, solange das Volumen des Balgs 5 noch verkleinert wird.
- Sobald der Balg 5 vollständig zusammengedrückt ist, wird kein Gas mehr aus der Messkammer 3 durch die Eingabe-Fluidverbindung hindurch aus der Messkammer 3 herausgepresst. Das Ventil 2, 13 ist geöffnet, und Gas kann aus dem Mundstück 30 durch die Eingabe-Fluidverbindung hindurch in die Messkammer 3 gesaugt werden oder fließen.

Sobald ein verschließendes Ereignis detektiert ist, werden die folgenden Schritte ausgelöst:
- Die Stange 4 wird wieder vom Probeneinlass 1 weggeschoben, bis der Ventilkörper 2 den Verschlusselement-Sitz 13 erreicht. Beispielsweise wird der Hubmagnet 7 wieder stromlos geschaltet, und die Feder verschiebt die Stange 4 vom Probeneinlass 1 weg.
- Sobald der Ventilkörper 2 den Verschlusselement-Sitz 13 erreicht, ist die Eingabe-Fluidverbindung 31, 15, Sp, 18 wieder geschlossen, und die Messkammer 3 ist vom Mundstück 30 und von der Umgebung fluiddicht getrennt.
- Das Verschieben der Stange 4 vom Probeneinlass 1 weg bewirkt außerdem, dass die Platte 6 den Balg 5 auseinanderzieht. Das Auseinanderziehen des Balgs 5 erzeugt einen Unterdruck. Der Unterdruck bewirkt, dass Gas aus dem Mundstück 30 durch den Hohlraum 31 im Probeneinlass 1 und die Eingabe-Fluidverbindung 31, 15, Sp, 18 im Verbindungsstück 16 hindurch in die Messkammer 3 gesaugt wird. Die Menge des Gases, die durch diesen Unterdruck in die Messkammer 3 gesaugt wird, gehört zur Messkammer-Probe Pr.
- Das Verschieben der Stange 4 bewirkt weiterhin, dass das oder jedes optionale Mischelement auf der Stange 4 durch die Messkammer 3 bewegt wird und dadurch das Gas in der Messkammer 3 bis zu einem gewissen Grad durchmischt wird.
- Sobald die Platte 6 den Balg 5 vollständig auseinandergezogen hat, hat der Balg 5 sein maximales Volumen erreicht. Das Ventil 2, 13 ist wieder in der verschließenden Endposition angekommen.

Der elektrochemische Sensor 12 analysiert die Gasprobe in der Messkammer 3, beispielsweise so wie mit Bezug auf Figur 1 beschrieben. Hierbei oxidiert die Messelektrode 20 das in der Messkammer 3 befindliche Atemalkohol. Die Messkammer-Probe Pr befindet sich in der Messkammer 3, bis die Stange 4 wieder auf den Probeneinlass 1 zu geschoben wird und dadurch der Balg 5 zusammengedrückt wird. Die Zeitspanne zwischen
- dem Ereignis, dass der Balg 5 vollständig auseinandergezogen ist und das Ventil 2, 13 geschlossen ist, und
- dem Ereignis, dass begonnen wird, den Balg 5 wieder zusammenzudrücken,
steht dem elektrochemischen Sensor 12 für die Analyse, insbesondere für das Oxidieren, der Messkammer-Probe Pr zur Verfügung. In dieser Zeitspanne ist das Ventil 2, 13 geschlossen. In der Regel reicht diese Zeitspanne aus, um Alkohol in der Messkammer-Probe Pr vollständig zu oxidieren.

Die zweite Ausgestaltung gemäß Figur 9 weist folgende Abweichungen zu der ersten Ausgestaltung gemäß Figur 2 bis Figur 7 auf:
- Der Dichtring 13 befindet sich flussaufwärts von dem Dichtkegel 2, während sich bei der ersten Ausgestaltung der Dichtring 13 flussabwärts vom Dichtkegel 2 befindet.
- Im Ruhezustand hält die nicht gezeigte Feder die Stange 4 in einer Position, in der die Stange 4 den kleinstmöglichen Abstand vom Probeneinlass 30 aufweist.
- Der aktivierte Hubmagnet 7 zieht die Stange 4 gegen die Kraft der Feder vom Probeneinlass 1 weg.
- Das Verschieben der Stange 4 vom Probeneinlass 1 weg bewirkt, dass der Dichtkegel 2 vom Dichtring 13 weg und auf die Messkammer 3 zu bewegt wird und dadurch das Ventil 2, 13 geöffnet wird.
- Außerdem bewirkt das Verschieben der Stange 4 vom Probeneinlass 1 weg, dass die Platte 6 den Balg 5 auseinander zieht.
- Weil der Balg 5 auseinander gezogen wird, fließt Gas durch die Eingabe-Fluidverbindung 31, 15, Sp, 18 hindurch in die Messkammer 3. Das Gas, das dergestalt in die Messkammer 3 fließt, gehört zur Messkammer-Probe Pr.
- Das verschließende Ereignis löst den Schritt aus, dass die Stange 4 wieder auf den Probeneinlass 1 hin geschoben wird, beispielsweise durch die Feder.
- Der Dichtkegel 2 wird von der Messkammer 3 weg und auf den Dichtring 13 zu bewegt. Sobald der Dichtkegel 2 den Dichtring 13 erreicht hat, ist das Ventil 2, 13 wieder geschlossen.
- Das Verschieben der Stange 4 auf den Probeneinlass 1 zu bewirkt außerdem, dass die Platte 6 den Balg 5 wieder zusammenschiebt. Das Zusammenschieben des Balgs 5 bewirkt einen Überdruck. Dieser Überdruck bewirkt, dass Gas und damit die Messkammer-Probe Pr aus dem Balg 5 durch die Ansaug-Fluidverbindung 8 hindurch in die Messkammer 3 geschoben wird. Dadurch wird Gas aus der Messkammer 3 durch die Eingabe-Fluidverbindung 15, Sp, 18 und durch den Hohlraum 31 hindurch in den Probeneinlass 1 hineingeschoben. Dadurch wird die Messkammer 3 gespült.

Bei der zweiten Ausgestaltung gemäß Figur 9 steht dem Sensor 12 folgende Zeitspanne zur Verfügung, um die Messkammer-Probe Pr zu analysieren: die Zeitspanne zwischen
- dem Ereignis, dass der Balg 5 vollständig auseinandergezogen ist, und
- dem Ereignis, dass das Zusammenschieben des Balgs 5 wieder begonnen wird.

In dieser Zeitspanne ist das Ventil 2, 13 vollständig geöffnet.

Damit der elektrochemische Sensor 12 ein Gas und damit auch die Messkammer-Probe Pr in der Messkammer 3 zuverlässig auf das Vorhandensein von Atemalkohol untersuchen kann und die Menge oder Konzentration von Atemalkohol in der Messkammer 3 messen kann, sollte wenigstens näherungsweise bekannt sein, welche Menge (Masse) der Atemprobe A sich bei der Analyse in der Messkammer 3 befindet, welche Menge also die Messkammer-Probe Pr aufweist. Durch die Konstruktion des Analysegeräts 100 ist das Volumen der Messkammer 3 bekannt. Die Differenz zwischen dem maximalem Volumen und dem minimalem Volumen des Balgs 5 ist ebenfalls durch die Konstruktion bekannt. Idealerweise fließt ausschließlich Luft aus der Lunge des Probanden in die Messkammer 3, aber keine Luft aus dem Mund und den oberen Atemwegen, sodass die Messkammer-Probe Pr nur aus Luft aus der Lunge besteht.

Bei beiden oben beschriebenen Ausgestaltungen wird dadurch Gas in die Messkammer 3 gesaugt, dass der Balg 5 auseinandergezogen und dadurch vom Zustand mit minimalem Volumen in den Zustand mit maximalem Volumen überführt wird. Die Differenz zwischen dem maximalem Volumen und dem minimalem Volumen des Balgs 5 ist in vielen Fällen gleich dem Volumen der Atemluft, die aus dem Mundstück 30 in die Messkammer 3 eingesaugt wird.

Außerdem kann während einer Zeitspanne, in der das Ventil 2, 13 geöffnet ist oder wird und gleichzeitig der Balg 5 nicht bewegt wird, Gas in die Messkammer 3 fließen, beispielsweise weil der Proband weiter ausatmet oder durch Diffusion. In vielen Fällen kann aber die Menge von Gas, die bei nicht bewegtem Balg 5 in die Messkammer 3 fließt, vernachlässigt werden.

In einer Ausgestaltung misst ein Drucksensor, der in einer Fluidverbindung mit der Messposition MP.1 steht, den zeitlichen Verlauf eines Maßes für den Überdruck im Mundstück 30 gegenüber dem Umgebungsdruck. Aus diesem zeitlichen Druckverlauf lässt sich in manchen Fällen herleiten, welches Volumen von Atemgas in einer Zeitspanne, in der das Ventil 2, 13 geöffnet ist oder wird und zugleich der Balg 5 nicht bewegt wird, in die Messkammer 3 fließt.

In einer Ausgestaltung misst ein Volumenfluss-Sensor die Differenz zwischen den Drücken an den beiden Messpunkten MP.1 und MP.2 und ermittelt hieraus den Volumenfluss. Durch Aufintegrieren über eine bestimmte Zeitspanne wird aus dem Volumenfluss das Volumen hergeleitet, das in dieser Zeitspanne durch die Eingabe-Fluidverbindung 31, 15, Sp, 18 hindurch in die Messkammer 3 fließt. Diese Zeitspanne ist beispielsweise gleich der Zeitspanne, in der das Ventil 2, 13 geöffnet ist und zugleich der Balg 5 nicht bewegt wird. Optional umfasst die Mess-Zeitspanne zusätzlich die Zeitspanne, in welcher der Ventilkörper 2 bewegt wird. Möglich ist auch, dass diese Mess-Zeitspanne zusätzlich die Zeitspanne umfasst, in welcher der Balg 5 auseinandergezogen wird, so dass mithilfe des Volumenfluss-Sensors auch gemessen wird, welches Volumen in die Messkammer 3 gesaugt wird.

Um eine gültige Atemprobe A abzugeben, muss der Proband bei dem gerade beschriebenen Ablauf mindestens so lange in das Mundstück 30 ausatmen und dadurch die Atemprobe A abgeben, bis der Balg 5 vollständig auseinandergezogen ist. Falls der Proband das Abgeben der Atemprobe A vorher abbricht, so wird bevorzugt eine entsprechende Meldung in einer von einem Menschen wahrnehmbaren Form ausgegeben. Bevorzugt kann der Proband dann eine weitere Atemprobe A abgeben.

Nachfolgend werden verschiedene Ausführungsformen beschrieben, wie das öffnende Ereignis festgelegt sein kann. Wie bereits erwähnt, wird dann damit begonnen, das Ventil 2, 13 in die freigebende Endposition zu bewegen, wenn das öffnende Ereignis detektiert ist. Idealerweise ist das öffnende Ereignis dann eingetreten, wenn die Luft aus der Lunge des Probanden das Mundstück 30 erreicht hat.
- In einer Ausgestaltung ist das öffnende Ereignis dann eingetreten, wenn seit dem Aufsetzen des Mundstücks 30 eine vorgegebene Zeitspanne verstrichen ist.
- In einer anderen Ausgestaltung wird näherungsweise gemessen, welche Menge von ausgeatmeter Luft der Proband in das Mundstück 30 eingegeben hat, seitdem das Mundstück 30 aufgesetzt wurde. Wie bereits dargelegt, misst in einer Ausgestaltung ein Drucksensor, der in einer Fluidverbindung mit dem ersten Messpunkt MP.1 steht, mehrmals hintereinander ein Maß für den Überdruck im Mundstück 30 relativ zum Umgebungsdruck. Aus den gemessenen Werten für die Druckdifferenz wird mindestens einmal ein Schätzwert für das bislang eingegebene Volumen hergeleitet. Wenn das bislang eingegebene Volumen eine vorgegebene Volumen-Schranke erreicht, ist das öffnende Ereignis eingetreten. Diese Volumen-Schranke ist bevorzugt gleich dem durchschnittlichen Volumen des Munds und der oberen Atemwege eines Erwachsenen.
- Eine weitere Ausgestaltung lässt sich insbesondere in Verbindung mit der oben beschriebenen und nicht gezeigten Ausgabe-Fluidverbindung anwenden, nämlich dann, wenn die Ausgabe-Fluidverbindung das Mundstück 30 mit der Umgebung verbindet. Solange das Ventil 2, 13 geschlossen ist, wird die Atemluft, die der Proband in das Mundstück 30 eingegeben hat, durch die Ausgabe-Fluidverbindung hindurch in die Umgebung geleitet. Der oben beschriebene Volumenfluss-Sensor mit den beiden Messpositionen MP.1 und MP.2 misst den Volumenfluss durch die Eingabe-Fluidverbindung 31, 15, Sp, 18 und die Ausgabe-Fluidverbindung hindurch. Aus dem gemessenen Volumenfluss wird das bislang eingegebene Volumen abgeleitet. Sobald das bislang eingegebene Volumen die Volumen-Schranke erreicht, ist das öffnende Ereignis eingetreten.

Der Schritt, die Bewegung des Ventils 2, 13 zurück in die verschließende Endposition zu beginnen, wird durch das verschließende Ereignis ausgelöst. Verschiedene Ausgestaltungen sind möglich, wann das verschließende Ereignis eingetreten ist:
- In einer Ausgestaltung ist das verschließende Ereignis eingetreten, sobald das Ventil 2, 13 die freigebende Endposition erreicht hat. Das Ventil 2, 13 bleibt also nur eine sehr kurze Zeitspanne, idealerweise nur einen Zeitpunkt lang, in der freigebenden Endposition. Gas wird ausschließlich dadurch in die Messkammer 3 gesaugt, dass der Balg 5 auseinandergezogen wird, also vom Zustand mit minimalem Volumen in den Zustand mit maximalem Volumen überführt wird. Die Menge, beispielsweise die Masse, der Messkammer-Probe Pr, welche in die Messkammer 3 gelangt, wird durch die Differenz zwischen dem maximalen Volumen und dem minimalen Volumen des Balgs 5 festgelegt.
- Bei einer anderen Ausgestaltung wird automatisch ermittelt, wann die chemische Reaktion in der Messkammer 3 beendet ist. Bei Beendigung der chemischen Reaktion ist alles Atemalkohol in der Messkammer 3 oxidiert. Um dieses Ereignis festzustellen, wird der zeitliche Verlauf des Signals, das der Sensor 12 erzeugt, ermittelt. Bleibt das Signal des Sensors 12 annähernd konstant, so ist der chemische Prozess abgeschlossen. Der Abschluss des chemischen Prozesses fungiert als das verschließende Ereignis.

Diese andere Ausgestaltung lässt sich insbesondere in Verbindung mit der zweiten Ausgestaltung (Figur 9) anwenden. Denn bei der zweiten Ausgestaltung bewirkt das verschließende Ereignis, dass der Balg 5 zusammengedrückt wird und die Messkammer-Probe Pr aus der Messkammer 3 herausgedrückt und dadurch die Messkammer 3 gespült wird. Bis dahin muss also die Analyse der Messkammer-Probe Pr abgeschlossen sein. Zuvor ist die Eingabe-Fluidverbindung hergestellt.

In den bislang beschriebenen Ausgestaltungen gelangt Atemluft, die idealerweise nur aus der Lunge des Probanden stammt, in die Messkammer 3 und fungiert dort als die zu analysierende Messkammer-Probe Pr. Möglich ist, dass zusätzlich vorab eine Vorprobe in die Messkammer 3 gesaugt und wieder aus der Messkammer 3 ausgestoßen wird, bevor Luft aus der Lunge des Probanden in die Messkammer 3 gerät. Der Balg 5 wird also zweimal auseinandergezogen und wieder zusammengedrückt, um denselben Probanden auf Alkohol zu untersuchen. In der Regel besteht diese Vorprobe überwiegend aus Luft, die aus dem Mund und / oder den oberen Atemwegen des Probanden stammt. Mithilfe der Vorprobe wird die Messkammer 3 ausgespült. In einer Ausgestaltung wird zusätzlich wenigstens näherungsweise ermittelt, wie groß der Gehalt an Atemalkohol im Mund und / oder in den oberen Atemwegen des Probanden ist. In einer anderen Ausgestaltung wird die Vorprobe verwendet, um die Elektroden 20, 21 des Sensors 12 auf die Temperatur der Atemprobe A zu bringen. In der Regel hat die Atemprobe A eine höhere Temperatur als die Umgebungsluft. Diese Ausgestaltung vergrößert in manchen Fällen die Zuverlässigkeit des Messergebnisses. Die beiden gerade beschriebenen Ausgestaltungen lassen sich miteinander kombinieren.

Der Balg 5 steht über die Ansaug-Fluidverbindung 8 in einer Fluidverbindung mit der Messkammer 3. Der Vorgang, den Balg 5 zusammenzudrücken, bewirkt, dass Gas durch die Ansaug-Fluidverbindung 8 in die Messkammer 3 gepresst wird und dadurch die Messkammer 3 gespült wird. In den bislang beschriebenen und in den Figuren gezeigten Ausgestaltungen wird das Gas, das aus der Messkammer 3 herausgepresst wird, durch die Eingabe-Fluidverbindung 31, 15, Sp, 18 in das Mundstück 30 gepresst.

In einer abweichenden und nicht gezeigten Ausgestaltung umfasst das Analysegerät zusätzlich eine Auslass-Fluidverbindung. Die Messkammer 3 steht über diese Auslass-Fluidverbindung in einer Fluidverbindung mit der Umgebung.

Ein Dreiwege-Ventil lässt sich wahlweise in einer von folgenden drei Positionen bringen:
- in eine Einlass-Position, in der das Dreiwege-Ventil die Eingabe-Fluidverbindung 31, 31, 15, Sp, 18 freigibt und zugleich die Auslass-Fluidverbindung versperrt,
- in eine Auslass-Position, in der das Dreiwege-Ventil die Auslass-Fluidverbindung freigibt und zugleich die Eingabe-Fluidverbindung 31, 31, 15, Sp, 18 versperrt, und
- optional in eine Sperr-Position, in der das Dreiwege-Ventil beide Fluidverbindungen versperrt.

Während des Vorgangs, den Balg 5 auseinander zu ziehen, ist das Dreiwege-Ventil in der Einlass-Position, sodass Gas durch die Eingabe-Fluidverbindung 31, 15, Sp, 18 hindurch in die Messkammer 3 strömen kann. Während des Vorgangs, den Balg 5 zusammenzudrücken, ist das Dreiwege-Ventil in der Auslass-Position, sodass Gas durch die Auslass-Fluidverbindung hindurch aus der Messkammer 3 herausfließen kann. Diese Ausgestaltung führt dazu, dass beim Spülen der Messkammer 3 das Gas in die Umgebung und nicht in das Mundstück 3 ausgestoßen wird. Bevorzugt ist das Dreiwege-Ventil in der Sperr-Position, während der Balg 5 nicht bewegt wird.

In einer Ausgestaltung bewirkt das öffnende Ereignis, dass das Dreiwege-Ventil in die Einlass-Position bewegt wird. In einer bevorzugten Ausgestaltung bewirkt das verschließende Ereignis, dass das Dreiwege-Ventil in die Auslass-Position bewegt wird.

### Bezugszeichenliste

| | |
|---|---|
| 1 | Probeneinlass, umgibt die Röhre 31, gehört zur Eingabe-Fluidführungseinheit |
| 2 | linear beweglicher Dichtkegel, fungiert als Ventilkörper und als Verschlussteil, relativ zum Ventilkörper-Sitz 13 beweglich, flussaufwärts (erste Ausgestaltung) bzw. flussabwärts (zweite Ausgestaltung) vom Ventilkörper-Sitz 13 angeordnet |
| 3 | Messkammer, nimmt eine Probe auf, die durch den Probeneinlass 1 einströmt, umgibt den Sensor 12, ist von der Wandung 40 umgeben |
| 4 | Stange, verbindet den Dichtkegel 2 mit dem Hubmagneten 7, durch die Verbindungs-Hülse 11 und die Messkammer 3 hindurch geführt, geführt, gehört zum mechanischen Verbindungselement |
| 5 | Balg, der einen Unterdruck und einen Überdruck in der Messkammer 3 zu erzeugen vermag, wird von der Platte 6 auseinandergezogen und zusammengedrückt, fungiert als Ansaug-Kammer |
| 6 | Platte, vermag den Balg 5 auseinanderzuziehen und zusammenzudrücken, fungiert als Kammer-Veränderungselement |
| 7 | Hubmagnet, bewegt linear die Stange 4 parallel zu deren Längsachse, Längsachse, fungiert als Stellantrieb |
| 8 | Ansaug-Fluidverbindung zwischen der Messkammer 3 und dem Balg 5 |
| 9 | Gestell, auf dem der Probeneinlass 1, die Wandung 40, der Sensor 12 und der Hubmagnet 7 befestigt sind |
| 10 | ausströmseitiges Anschlussstück, an der Messkammer 3 befestigt |
| 11 | Verbindungs-Hülse, durch welche hindurch die Stange 4 geführt ist, fest mit der Stange 4 und mit der Platte 6 verbunden, gehört zum mechanischen Verbindungselement |
| 12 | elektrochemischer Sensor in der Messkammer 3, umfasst die Elektroden 20 und 21 sowie die elektrischen Kontaktierungen 33, 34, vermag ein Maß für die Konzentration von Atemalkohol in der Messkammer-Probe Pr zu ermitteln |
| 13 | Dichtring um die Stange 4, fungiert als Ventilkörper-Sitz und somit als Verschlussteil-Sitz für den Dichtkegel (Ventilkörper) 2, flussabwärts (erste Ausgestaltung) bzw. flussaufwärts (zweite Ausgestaltung) vom Dichtkegel 2 angeordnet |
| 14 | weiterer Dichtring, um die Röhre 16 herum angeordnet |
| 15 | Hohlraum im Verbindungsstück 16 |
| 16 | Verbindungsstück zwischen dem Probeneinlass 1 und dem Dichtkegel 2, umgibt den Hohlraum 15, umfasst die Teile 16.1 und 16.2, gehört zur Eingabe-Fluidführungseinheit |
| 16.1 | kleinerer Teil des Verbindungsstücks 16 |
| 16.2 | größerer Teil des Verbindungsstücks 16 |
| 17 | Deckplatte für den Sensor 12 |
| 18 | Aussparung, die zu einer Eingabe-Fluidverbindung zwischen dem Hohlraum 15 und der Messkammer 3 gehört |
| 19 | Führungseinheit, welche die Stange 4 linear führt |
| 20 | Messelektrode des Sensors 12, wird von der elektrischen Kontaktierung 34 kontaktiert |
| 21 | Gegenelektrode des Sensors 12, wird von der elektrischen Kontaktierung 33 kontaktiert |
| 22 | elektrische Verbindung zwischen den Kontaktierungen 33 und 34 |
| 25 | Anschlagelement auf dem Probeneinlass 1, begrenzt eine Bewegung des Mundstücks 30 auf die Messkammer 3 zu |
| 26 | Verbindungselement zwischen der Platte 6 und dem Hubmagneten 7 |
| 27 | Bolzen, durch eine Aussparung im Verbindungselement 26 und in einer Stange des Hubelementen 7 hindurchgeführt |
| 28 | Elektrolyt zwischen den beiden Elektroden 20 und 21 |
| 29 | elektrischer Messwiderstand zwischen den beiden Elektroden 20, 21 |
| 30 | trichterförmiges Mundstück, leitet eine Atemprobe A in den Probeneinlass 1 |
| 31 | Röhre im Inneren des Probeneinlasses 1 |
| 32 | einströmseitiges Anschlussstück, an der Messkammer 3 befestigt, umgibt das größere Teil 16.2 |
| 33 | elektrische Kontaktierung der Gegenelektrode 21 |
| 34 | elektrische Kontaktierung der Messelektrode 20 |
| 38 | Stromstärken-Sensor, misst die Stärke des durch die elektrische Verbindung 22 fließenden Stroms |
| 40 | Wandung der Messkammer 3 |
| 50 | Sensor-Anordnung, umfasst einen Sensor 12 und eine Messkammer 3 |
| 60 | Steuergerät, steuert den Hubmagneten 7 an |
| 100 | Analysegerät, umfasst das Mundstück 30, das Gestell 9, den Probeneinlass 1, die Messkammer 3, den Sensor 12, die Stange 4, das Ventil 2, 13, den Stellantrieb mit dem Hubmagneten 7 und der Verbindungs-Hülse 11 |
| A | Atemprobe, die auf Atemalkohol zu untersuchen ist, enthält die Messkammer-Probe Pr, die in die Messkammer 3 gesaugt wird |
| MA | übereinstimmende Mittelachse der Messkammer 3 und des Sensors 12 |
| MP.1 | erster Messpunkt, steht in einer Fluidverbindung mit dem Hohlraum 15 oder der Aussparung 18 und dadurch in einer Fluidverbindung mit dem Mundstück 30 |
| MP.2 | zweiter Messpunkt, steht in einer Fluidverbindung mit der Messkammer 3 |
| Ö.a | austrittseitige Öffnung im Gehäuse 11, durch welche hindurch die Messkammer-Probe Pr aus der Messkammer 3 heraus fließt |
| Ö.e | eintrittsseitige Öffnung im Gehäuse 11, durch welche hindurch die Messkammer-Probe Pr in die Messkammer 3 hinein fließt |
| Pr | Messkammer-Probe, das ist derjenige Teil der vom Probanden abgegebene Atemprobe A, die in die Messkammer 3 gelangt |
| Sp | umlaufender Spalt zwischen dem Dichtkegel 2 und der Innenwand des Hohlraums 15 |

## Patentansprüche

1. Analysegerät (100) zum Analysieren einer von einem Probanden abgegebenen, insbesondere ausgeatmeten, Gasprobe (A) auf eine vorgegebene Substanz, insbesondere auf Alkohol,
wobei das Analysegerät (100)
- eine Eingabeeinheit (30) zum Eingeben oder Aufnehmen der Gasprobe (A),
- eine Messkammer (3),
- einen Sensor (12),
- eine Ansaug-Kammereinheit (5, 6), die wahlweise in einen Zustand mit minimalem Volumen und einen Zustand mit maximalem Volumen überführbar ist,
- ein Ventil (2, 13) und
- eine Antriebseinheit (7, 4, 11)
umfasst,
wobei das Analysegerät (100)
- wenigstens zeitweise eine Eingabe-Fluidverbindung (31, 15, Sp, 18) zwischen der Eingabeeinheit (30) und der Messkammer (3) und
- wenigstens zeitweise eine Ansaug-Fluidverbindung (8) zwischen der Ansaug-Kammereinheit (5, 6) und der Messkammer (3)
bereitstellt,
wobei das Analysegerät (100) so ausgestaltet ist, dass eine Überführung der Ansaug-Kammereinheit (5, 6) in den Zustand mit maximalem Volumen bewirkt, dass Gas aus der Eingabeeinheit (30) durch die Eingabe-Fluidverbindung (31, 15, Sp, 18) hindurch in die Messkammer (3) gesaugt wird,
wobei der Sensor (12) dazu ausgestaltet ist, ein Maß für die Menge und / oder die Konzentration der Substanz in einem Gas (Pr), das sich in der Messkammer (3) befindet, zu messen,
wobei die Antriebseinheit (7, 4, 11) dazu ausgestaltet ist, das Ventil (2, 13) wahlweise
- in eine verschließende Endposition, in der das Ventil (2, 13) die Eingabe-Fluidverbindung (31, 15, Sp, 18) unterbricht, oder
- in eine freigebende Endposition, in der das Ventil (2, 13) die Eingabe-Fluidverbindung (31, 15, Sp, 18) freigibt,
zu bewegen,
wobei die Antriebseinheit (7, 4, 11) weiterhin dazu ausgestaltet ist, die Ansaug-Kammereinheit (5, 6) wahlweise in den Zustand mit minimalem Volumen oder in den Zustand mit maximalem Volumen zu überführen,
wobei die Antriebseinheit (7, 4, 11) dergestalt mit dem Ventil (2, 13) und mit der Ansaug-Kammereinheit (5, 6) mechanisch gekoppelt ist, dass entweder
- eine Bewegung des Ventils (2, 13) in die freigebende Endposition mit einer Überführung der Ansaug-Kammereinheit (5, 6) in den Zustand mit minimalem Volumen synchronisiert ist und
- eine Bewegung des Ventils (2, 13) in die verschließende Endposition mit einer Überführung der Ansaug-Kammereinheit (5, 6) in den Zustand mit maximalem Volumen synchronisiert ist
oder
- eine Bewegung des Ventils (2, 13) in die freigebende Endposition mit einer Überführung der Ansaug-Kammereinheit (5, 6) in den Zustand mit maximalem Volumen synchronisiert ist und
- eine Bewegung des Ventils (2, 13) in die verschließende Endposition mit einer Überführung der Ansaug-Kammereinheit (5, 6) in den Zustand mit minimalem Volumen synchronisiert ist.

2. Analysegerät (100) nach Anspruch 1,
**dadurch gekennzeichnet, dass**
die Antriebseinheit (7, 4, 11)
- einen Stellantrieb (7) und
- ein mechanisches Ventil-Verbindungselement (4, 11)
umfasst und
das Ventil (2, 13)
- ein Verschlussteil (2) und
- einen Verschlussteil-Sitz (13)
umfasst,
wobei das Ventil-Verbindungselement (4, 11) den Stellantrieb (7) mit dem Verschlussteil (2) mechanisch verbindet.

3. Analysegerät (100) nach einem der vorhergehenden Ansprüche,
**dadurch gekennzeichnet, dass**
die Messkammer (3) sich zwischen der Eingabeeinheit (30) und der Ansaug-Kammereinheit (5, 6) befindet.

4. Analysegerät (100) nach einem der vorhergehenden Ansprüche,
**dadurch gekennzeichnet, dass**
die Antriebseinheit (7, 4, 11) einen Stellantrieb (7) umfasst, der mit dem Ventil (2, 13) und der Ansaug-Kammereinheit (5, 6) mechanisch gekoppelt ist, und
die Ansaug-Kammereinheit (5, 6) sich zwischen der Messkammer (3) und dem Stellantrieb (7) befindet.

5. Analysegerät (100) nach einem der vorhergehenden Ansprüche,
**dadurch gekennzeichnet, dass**
die Antriebseinheit (7, 4, 11)
- einen Stellantrieb (7) und
- ein mechanisches Kammer-Verbindungselement (4, 11)
umfasst und
die Ansaug-Kammereinheit (5, 6)
- eine Ansaug-Kammer (5) mit veränderbarem Volumen und
- ein Kammer-Veränderungselement (6)
umfasst,
wobei die Ansaug-Fluidverbindung (8) die Ansaug-Kammer (5) mit der Messkammer (3) verbindet,
wobei eine Bewegung des Kammer-Veränderungselements (6) relativ zur Ansaug-Kammer (5) bewirkt, dass das Volumen der Ansaug-Kammer (5) verändert wird, und
wobei das Kammer-Verbindungselement (4, 11) den Stellantrieb (7) mit dem Kammer-Veränderungselement (6) mechanisch verbindet.

6. Analysegerät (100) nach Anspruch 2 und nach Anspruch 5,
**dadurch gekennzeichnet, dass**
das Analysegerät (100) ein mechanisches Verbindungselement (4, 11) umfasst,
wobei das mechanische Verbindungselement (4, 11) sowohl das Ventil-Verbindungselement als auch das Kammer-Verbindungselement umfasst oder bereitstellt.

7. Analysegerät (100) nach einem der vorhergehenden Ansprüche,
**dadurch gekennzeichnet, dass**
das Analysegerät (100) einen Volumenfluss-Sensor (MP.1, MP.2) umfasst,
wobei der Volumenfluss-Sensor (MP.1, MP.2) dazu ausgestaltet ist, ein Maß für den Volumenfluss eines Gases (Pr) durch die Eingabe-Fluidverbindung (31, 15, Sp, 18) hindurch in die Messkammer (3) zu messen, und
wobei das Analysegerät (100) dazu ausgestaltet ist, automatisch und abhängig vom gemessenen Volumenfluss den Schritt auszulösen,
dass die Antriebseinheit (7, 4, 11) das Ventil (2, 13) abhängig vom gemessenen Volumenfluss in die verschließende Endposition bewegt.

8. Analysegerät (100) nach einem der vorhergehenden Ansprüche,
**dadurch gekennzeichnet, dass**
das Ventil (2, 13) ein Verschlussteil (2) umfasst und
das Analysegerät (100) eine Fluidführungseinheit (16) umfasst, die das Verschlussteil (2) umgibt,
wobei zwischen der Fluidführungseinheit (16) und dem Verschlussteil (2) ein Zwischenraum (Sp) auftritt und
wobei die Eingabe-Fluidverbindung (31, 15, Sp, 18)
- durch die Fluidführungseinheit (16) hindurch führt und
- den Zwischenraum (Sp) umfasst.

9. Analysegerät (100) nach einem der vorhergehenden Ansprüche,
**dadurch gekennzeichnet, dass**
das Analysegerät (100) so ausgestaltet ist,
dass eine Überführung der Ansaug-Kammereinheit (5, 6) in den Zustand mit minimalem Volumen bewirkt,
dass Gas durch die Eingabe-Fluidverbindung (31, 15, Sp, 18) hindurch aus der Messkammer (3) hinaus gefördert wird.

10. Analysegerät (100) nach einem der vorhergehenden Ansprüche,
**dadurch gekennzeichnet, dass**
das Analysegerät (100) eine Eingabe-Fluidführungseinheit (1, 16, 40) umfasst,
wobei die Eingabeeinheit (30) sich mit der Eingabe-Fluidführungseinheit (1, 16, 40) verbinden lässt, bevorzugt lösbar verbinden lässt,
wobei die Eingabe-Fluidverbindung (31, 15, Sp, 18) durch die Eingabe-Fluidführungseinheit (1, 16, 40) hindurchführt und
wobei die Eingabe-Fluidführungseinheit (1, 16, 40) das Ventil (2, 13) umgibt, bevorzugt vollständig umgibt.

11. Verfahren zum Analysieren einer von einem Probanden abgegebenen, insbesondere ausgeatmeten, Gasprobe (A) auf eine vorgegebene Substanz, insbesondere auf Alkohol,
unter Verwendung eines Analysegeräts (100), das
- eine Eingabeeinheit (30),
- eine Messkammer (3),
- einen Sensor (12),
- eine Ansaug-Kammereinheit (5, 6), die wahlweise in einen Zustand mit minimalem Volumen und einen Zustand mit maximalem Volumen überführbar ist,
- ein Ventil (2, 13) und
- eine Antriebseinheit (7, 4, 11)
umfasst,
wobei das Analysegerät (100)
- wenigstens zeitweise eine Eingabe-Fluidverbindung (31, 15, Sp, 18) zwischen der Eingabeeinheit (30) und der Messkammer (3) und
- wenigstens zeitweise eine Ansaug-Fluidverbindung (8) zwischen der Ansaug-Kammereinheit (5, 6) und der Messkammer (3) bereitstellt,
wobei vor der Durchführung des Verfahrens das Ventil (2, 13) in einer verschließenden Endposition ist, in der das Ventil (2, 13) die Eingabe-Fluidverbindung (31, 15, Sp, 18) unterbricht, und
wobei das Verfahren die Schritte umfasst, dass
- die Gasprobe (A) in die Eingabeeinheit (30) eingegeben und / oder von der Eingabeeinheit (30) aufgenommen wird,
- die Antriebseinheit (7, 4, 11) das Ventil (2, 13) in eine freigebende Endposition bewegt, in der das Ventil (2, 13) die Eingabe-Fluidverbindung (31, 15, Sp, 18) freigibt,
- die Antriebseinheit (7, 4, 11) die Ansaug-Kammereinheit (5, 6) in den Zustand mit maximalem Volumen überführt, sodass Gas aus der Eingabeeinheit (30) durch die Eingabe-Fluidverbindung (31, 15, Sp, 18) hindurch in die Messkammer (3) hinein gesaugt wird,
- die Antriebseinheit (7, 4, 11) die Ansaug-Kammereinheit (5, 6) anschließend in den Zustand mit minimalem Volumen überführt, sodass Gas aus der Ansaug-Kammereinheit (5, 6) durch die Ansaug-Fluidverbindung (8) hindurch in die Messkammer (3) gefördert wird und dadurch Gas aus der Messkammer (3) ausgestoßen wird und
- die Antriebseinheit (7, 4, 11) das Ventil (2, 13) wieder in die verschließende Endposition bewegt,
wobei das Verfahren den weiteren Schritt umfasst, dass der Sensor (12) ein Maß für die Konzentration und / oder Menge der Substanz in dem Gas (Pr), das sich in der Messkammer (3) befindet, misst,
wobei
entweder
- der Schritt, das Ventil (2, 13) in die freigebende Endposition zu bewegen, und der Schritt, die Ansaug-Kammereinheit (5, 6) in den Zustand mit minimalem Volumen zu überführen, gleichzeitig durchgeführt werden, und
- der Schritt, das Ventil (2, 13) wieder in die verschließende Endposition zu bewegen, und der Schritt, die Ansaug-Kammereinheit (5, 6) in den Zustand mit maximalem Volumen zu überführen, gleichzeitig durchgeführt werden,
oder
- der Schritt, das Ventil (2, 13) in die verschließende Endposition zu bewegen, und der Schritt, die Ansaug-Kammereinheit (5, 6) in den Zustand mit minimalem Volumen zu überführen, gleichzeitig durchgeführt werden, und
- der Schritt, das Ventil (2, 13) wieder in die freigebende Endposition zu bewegen, und der Schritt, die Ansaug-Kammereinheit (5, 6) in den Zustand mit maximalem Volumen zu überführen, gleichzeitig durchgeführt werden.

12. Verfahren nach Anspruch 11,
**dadurch gekennzeichnet, dass**
automatisch das Ereignis detektiert wird, dass die Eingabe der Gasprobe (A) in die Eingabeeinheit (30) begonnen wird, und
der Schritt, das Ventil (2, 13) in die freigebende Endposition zu bewegen, begonnen wird, wenn
- seit dem Beginn der Eingabe der Gasprobe (A) eine vorgegebene Zeitspanne verstrichen ist und / oder
- nach dem Beginn der Eingabe der Gasprobe (A) ein öffnendes Ereignis eingetreten ist,
wobei das öffnende Ereignis von einem Maß für das Volumen und / oder der Menge der bislang in die Eingabeeinheit (30) eingegebenen Gasprobe (A) abhängt.

13. Verfahren nach Anspruch 11 oder Anspruch 12,
**dadurch gekennzeichnet, dass**
mindestens einmal ein Maß für die Menge von Gas, die nach dem Beginn des Schritts, das Ventil (2, 13) in die freigebende Endposition zu bewegen, bislang in die Messkammer (3) geflossen ist, gemessen wird und
dann, wenn die gemessene Menge eine vorgegebene Mengen-Schranke erreicht hat, automatisch der Schritt ausgelöst wird, das Ventil (2, 13) wieder in die verschließende Endposition zu bewegen.

14. Verfahren nach einem der Ansprüche 11 bis 13,
**dadurch gekennzeichnet, dass**
vor der Durchführung des Verfahrens die Ansaug-Kammereinheit (5, 6) in dem Zustand mit maximalem Volumen ist und
der Schritt, die Ansaug-Kammereinheit (5, 6) in den Zustand mit minimalem Volumen zu überführen,
vor dem Schritt, die Ansaug-Kammereinheit (5, 6) in den Zustand mit maximalem Volumen zu überführen, durchgeführt wird.

15. Verfahren nach einem der Ansprüche 11 bis 13,
**dadurch gekennzeichnet, dass**
vor der Durchführung des Verfahrens die Ansaug-Kammereinheit (5, 6) in dem Zustand mit minimalem Volumen ist und
der Schritt, die Ansaug-Kammereinheit (5, 6) in den Zustand mit maximalem Volumen zu überführen,
vor dem Schritt, die Ansaug-Kammereinheit (5, 6) in den Zustand mit minimalem Volumen zu überführen, durchgeführt wird.

16. Verfahren nach einem der Ansprüche 11 bis 15,
**dadurch gekennzeichnet, dass**
die Antriebseinheit (7, 4, 11) die Schritte,
- das Ventil in die eine Endposition zu bewegen und
- die Ansaug-Kammereinheit (5, 6) in den Zustand mit minimalem oder maximalem Volumen zu überführen,
mithilfe einer eine mechanische Koppelung der Antriebseinheit (7, 4, 11) mit dem Ventil (2, 13) und mit der Ansaug-Kammereinheit (5, 6) bewirkt.

## Claims

1. An analyzer (100) for analyzing a gas sample (A) emitted, in particular exhaled, by a test subject for a predetermined substance, in particular for alcohol,
wherein the analyzer (100) comprises
- an input unit (30) for inputting or receiving the gas sample (A),
- a measuring chamber (3),
- a sensor (12),
- a suction chamber unit (5, 6) which can be selectively transferred into a minimum volume state and a maximum volume state,
- a valve (2, 13), and
- a drive unit (7, 4, 11),
wherein the analyzer (100) provides
- an input fluid connection (31, 15, Sp, 18) between the input unit (30) and the measuring chamber (3) at least temporarily and
- a suction fluid connection (8) between the suction chamber unit (5, 6) and the measuring chamber (3) at least temporarily,
wherein the analyzer (100) is designed such that a transfer of the suction chamber unit (5, 6) into the maximum volume state causes gas to be sucked from the input unit (30) through the input fluid connection (31, 15, Sp, 18) into the measuring chamber (3),
wherein the sensor (12) is designed to measure an indicator of the amount and/or the concentration of the substance in a gas (Pr) located in the measuring chamber (3),
wherein the drive unit (7, 4, 11) is designed to selectively move the valve (2, 13)
- into a closing end position in which the valve (2, 13) interrupts the input fluid connection (31, 15, Sp, 18), or
- into a releasing end position in which the valve (2, 13) releases the input fluid connection (31, 15, Sp, 18),
wherein the drive unit (7, 4, 11) is further designed to selectively transfer the suction chamber unit (5, 6) into the minimum volume state or into the maximum volume state,
wherein the drive unit (7, 4, 11) is mechanically coupled to the valve (2, 13) and to the suction chamber unit (5, 6) in such a way that
either
- a movement of the valve (2, 13) into the releasing end position is synchronized with a transfer of the suction chamber unit (5, 6) into the minimum volume state and
- a movement of the valve (2, 13) into the closing end position is synchronized with a transfer of the suction chamber unit (5, 6) into the maximum volume state
or
- a movement of the valve (2, 13) into the releasing end position is synchronized with a transfer of the suction chamber unit (5, 6) into the maximum volume state and
- a movement of the valve (2, 13) into the closing end position is synchronized with a transfer of the suction chamber unit (5, 6) into the minimum volume state.

2. The analyzer (100) according to claim 1,
**characterized in that**
the drive unit (7, 4, 11) comprises
- an actuator (7) and
- a mechanical valve connecting element (4, 11)
and
the valve (2, 13) comprises
- a closure part (2) and
- a closure-part seat (13),
the valve connecting element (4, 11) mechanically connecting the actuator (7) to the closure part (2).

3. The analyzer (100) according to one of the preceding claims,
**characterized in that**
the measuring chamber (3) is located between the input unit (30) and the suction chamber unit (5, 6).

4. The analyzer (100) according to one of the preceding claims,
**characterized in that**
the drive unit (7, 4, 11) comprises an actuator (7) which is mechanically coupled to the valve (2, 13) and to the suction chamber unit (5, 6), and
the suction chamber unit (5, 6) is located between the measuring chamber (3) and the actuator (7).

5. The analyzer (100) according to one of the preceding claims,
**characterized in that**
the drive unit (7, 4, 11) comprises
- an actuator (7) and
- a mechanical chamber connecting element (4, 11)
and
the suction chamber unit (5, 6) comprises
- a suction chamber (5) with variable volume and
- a chamber modifying element (6),
the suction fluid connection (8) connecting the suction chamber (5) to the measuring chamber (3),
a movement of the chamber modifying element (6) relative to the suction chamber (5) causing the volume of the suction chamber (5) to be varied, and
the chamber connecting element (4, 11) mechanically connecting the actuator (7) to the chamber modifying element (6).

6. The analyzer (100) according to claim 2 and according to claim 5,
**characterized in that**
the analyzer (100) comprises a mechanical connecting element (4, 11),
the mechanical connecting element (4, 11) comprising or providing both the valve connecting element and the chamber connecting element.

7. The analyzer (100) according to one of the preceding claims,
**characterized in that**
the analyzer (100) comprises a volume flow rate sensor (MP.1, MP.2), the volume flow rate sensor (MP.1, MP.2) being designed to measure an indicator of the volume flow rate of a gas (Pr) through the input fluid connection (31, 15, Sp, 18) into the measuring chamber (3), and
the analyzer (100) being designed to trigger, automatically and depending on the measured volume flow rate, the step
whereby the drive unit (7, 4, 11) moves the valve (2, 13) into the closing end position depending on the measured volume flow rate.

8. The analyzer (100) according to one of the preceding claims,
**characterized in that**
the valve (2, 13) comprises a closure part (2) and
the analyzer (100) comprises a fluid guide unit (16) which surrounds the closure part (2),
a space (Sp) occurring between the fluid guide unit (16) and the closure part (2) and
the input fluid connection (31, 15, Sp, 18)
- passing through the fluid guide unit (16) and
- comprising the space (Sp).

9. The analyzer (100) according to one of the preceding claims,
**characterized in that**
the analyzer (100) is designed
such that a transfer of the suction chamber unit (5, 6) into the minimum volume state causes
gas to be conveyed out of the measuring chamber (3) through the input fluid connection (31, 15, Sp, 18).

10. The analyzer (100) according to one of the preceding claims,
**characterized in that**
the analyzer (100) comprises an input fluid guide unit (1, 16, 40),
it being possible for the input unit (30) to be connected, preferably detachably connected, to the input fluid guide unit (1, 16, 40),
the input fluid connection (31, 15, Sp, 18) passing through the input fluid guide unit (1, 16, 40) and
the input fluid guide unit (1, 16, 40) surrounding, preferably completely surrounding, the valve (2, 13).

11. A method for analyzing a gas sample (A) emitted, in particular exhaled, by a test person for a predetermined substance, in particular for alcohol,
using an analyzer (100) which comprises
- an input unit (30),
- a measuring chamber (3),
- a sensor (12),
- a suction chamber unit (5, 6) which can be selectively transferred into a minimum volume state and a maximum volume state,
- a valve (2, 13), and
- a drive unit (7, 4, 11),
wherein the analyzer (100) provides
- an input fluid connection (31, 15, Sp, 18) between the input unit (30) and the measuring chamber (3) at least temporarily and
- a suction fluid connection (8) between the suction chamber unit (5, 6) and the measuring chamber (3) at least temporarily,
wherein, before the method is carried out, the valve (2, 13) is in a closing end position in which the valve (2, 13) interrupts the input fluid connection (31, 15, Sp, 18), and
wherein the method comprises the steps whereby
- the gas sample (A) is input into the input unit (30) and/or received by the input unit (30),
- the drive unit (7, 4, 11) moves the valve (2, 13) into a releasing end position in which the valve (2, 13) releases the input fluid connection (31, 15, Sp, 18),
- the drive unit (7, 4, 11) transfers the suction chamber unit (5, 6) into the maximum volume state so that gas is sucked from the input unit (30) through the input fluid connection (31, 15, Sp, 18) into the measuring chamber (3),
- the drive unit (7, 4, 11) subsequently transfers the suction chamber unit (5, 6) into the minimum volume state so that gas is conveyed from the suction chamber unit (5, 6) through the suction fluid connection (8) into the measuring chamber (3) and thereby gas is expelled from the measuring chamber (3) and
- the drive unit (7, 4, 11) moves the valve (2, 13) back into the closing end position,
wherein the method comprises the further step whereby the sensor (12) measures an indicator of the concentration and/or amount of the substance in the gas (Pr) located in the measuring chamber (3),
wherein
either
- the step of moving the valve (2, 13) into the releasing end position and the step of transferring the suction chamber unit (5, 6) into the minimum volume state are carried out simultaneously, and
- the step of moving the valve (2, 13) back into the closing end position and the step of transferring the suction chamber unit (5, 6) into the maximum volume state are carried out simultaneously,
or
- the step of moving the valve (2, 13) into the closing end position and the step of transferring the suction chamber unit (5, 6) into the minimum volume state are carried out simultaneously, and
- the step of moving the valve (2, 13) back into the releasing end position and the step of transferring the suction chamber unit (5, 6) into the maximum volume state are carried out simultaneously.

12. The method according to claim 11,
**characterized in that**
the event whereby the input of the gas sample (A) into the input unit (30) is started is automatically detected, and
the step of moving the valve (2, 13) to the releasing end position is started if
- a predetermined period of time has elapsed since the start of the input of the gas sample (A) and/or
- an opening event has occurred after the start of the input of the gas sample (A),
the opening event depending on an indicator of the volume and/or amount of the gas sample (A) previously input into the input unit (30).

13. The method according to claim 11 or claim 12,
**characterized in that**
an indicator of the amount of gas that has previously flowed into the measuring chamber (3) since the start of the step of moving the valve (2, 13) into the releasing end position is measured at least once, and
then, when the measured amount has reached a predetermined amount limit, the step of moving the valve (2, 13) back into the closing end position is automatically triggered.

14. The method according to one of claims 11 to 13,
**characterized in that**
before the method is carried out, the suction chamber unit (5, 6) is in the maximum volume state and
the step of transferring the suction chamber unit (5, 6) into the minimum volume state is carried out
before the step of transferring the suction chamber unit (5, 6) into the maximum volume state.

15. The method according to one of claims 11 to 13,
**characterized in that**
before the method is carried out, the suction chamber unit (5, 6) is in the minimum volume state and
the step of transferring the suction chamber unit (5, 6) into the maximum volume state is carried out
before the step of transferring the suction chamber unit (5, 6) into the minimum volume state.

16. The method according to one of claims 11 to 15,
**characterized in that**
the drive unit (7, 4, 11) performs the steps of
- moving the valve into one end position and
- transferring the suction chamber unit (5, 6) into the minimum or maximum volume state,
by means of a mechanical coupling of the drive unit (7, 4, 11) with the valve (2, 13) and with the suction chamber unit (5, 6).

## Revendications

1. Appareil d'analyse (100) pour l'analyse d'un échantillon de gaz (A) émis, en particulier expiré, par un sujet, à la recherche d'une substance prédéfinie, en particulier d'alcool,
dans lequel l'appareil d'analyse (100) comprend
- une unité d'entrée (30) pour l'entrée ou le prélèvement de l'échantillon de gaz (A),
- une chambre de mesure (3),
- un capteur (12),
- une unité à chambre d'aspiration (5, 6) pouvant passer sélectivement dans un état de volume minimal et dans un état de volume maximal,
- une soupape (2, 13), et
- une unité d'entraînement (7, 4, 11),
dans lequel l'appareil d'analyse (100) met à disposition
- au moins temporairement, un raccordement fluidique d'entrée (31, 15, Sp, 18) entre l'unité d'entrée (30) et la chambre de mesure (3), et
- au moins temporairement, un raccordement fluidique d'aspiration (8) entre l'unité à chambre d'aspiration (5, 6) et la chambre de mesure (3),
dans lequel l'appareil d'analyse (100) est configuré de sorte que le fait de faire passer l'unité à chambre d'aspiration (5, 6) dans l'état de volume maximal provoque l'aspiration du gaz, depuis l'unité d'entrée (30) et à travers le raccordement fluidique d'entrée (31, 15, Sp, 18), dans la chambre de mesure (3),
dans lequel le capteur (12) est configuré pour mesurer une mesure de la quantité et/ou de la concentration de la substance dans un gaz (Pr) se trouvant dans la chambre de mesure (3),
dans lequel l'unité d'entraînement (7, 4, 11) est configurée pour déplacer sélectivement la soupape (2, 13)
- dans une position finale de fermeture dans laquelle la soupape (2, 13) interrompt le raccordement fluidique d'entrée (31, 15, Sp, 18), ou
- dans une position finale de libération dans laquelle la soupape (2, 13) libère le raccordement fluidique d'entrée (31, 15, Sp, 18),,
dans lequel l'unité d'entraînement (7, 4, 11) est en outre configurée pour faire passer sélectivement l'unité à chambre d'aspiration (5, 6) dans l'état de volume minimal ou dans l'état de volume maximal,
dans lequel l'unité d'entraînement (7, 4, 11) est accouplée mécaniquement à la soupape (2, 13) et à l'unité à chambre d'aspiration (5, 6) de telle sorte que
soit
- un déplacement de la soupape (2, 13) en position finale de libération est synchronisé avec un passage de l'unité à chambre d'aspiration (5, 6) dans l'état de volume minimal, et
- un déplacement de la soupape (2, 13) en position finale de fermeture est synchronisé avec un passage de l'unité à chambre d'aspiration (5, 6) dans l'état de volume maximal
soit
- un déplacement de la soupape (2, 13) en position finale de libération est synchronisé avec un passage de l'unité à chambre d'aspiration (5, 6) dans l'état de volume maximal, et
- un déplacement de la soupape (2, 13) en position finale de fermeture est synchronisé avec un passage de l'unité à chambre d'aspiration (5, 6) dans l'état de volume minimal.

2. Appareil d'analyse (100) selon la revendication 1,
**caractérisé en ce que**
l'unité d'entraînement (7, 4, 11) comprend
- un mécanisme de commande (7), et
- un élément de liaison de soupape (4, 11) mécanique
et
la soupape (2, 13) comprend
- une pièce de fermeture (2), et
- un siège pour pièce de fermeture (13),
dans lequel l'élément de liaison de soupape (4, 11) relie mécaniquement le mécanisme de commande (7) à la pièce de fermeture (2).

3. Appareil d'analyse (100) selon l'une des revendications précédentes,
**caractérisé en ce que**
la chambre de mesure (3) se trouve entre l'unité d'entrée (30) et l'unité à chambre d'aspiration (5, 6).

4. Appareil d'analyse (100) selon l'une des revendications précédentes,
**caractérisé en ce que**
l'unité d'entraînement (7, 4, 11) comprend un mécanisme de commande (7) accouplé mécaniquement à la soupape (2, 13) et à l'unité à chambre d'aspiration (5, 6), et
l'unité à chambre d'aspiration (5, 6) se trouve entre la chambre de mesure (3) et le mécanisme de commande (7).

5. Appareil d'analyse (100) selon l'une des revendications précédentes,
**caractérisé en ce que**
l'unité d'entraînement (7, 4, 11) comprend
- un mécanisme de commande (7), et
- un élément de liaison de chambre (4, 11) mécanique
et
l'unité à chambre d'aspiration (5, 6) comprend
- une chambre d'aspiration (5) à volume variable, et
- un élément de variation de chambre (6),
dans lequel le raccordement fluidique d'aspiration (8) raccorde la chambre d'aspiration (5) à la chambre de mesure (3),
dans lequel un déplacement de l'élément de variation de chambre (6) par rapport à la chambre d'aspiration (5) provoque une variation du volume de la chambre d'aspiration (5), et
dans lequel l'élément de liaison de chambre (4, 11) relie mécaniquement le mécanisme de commande (7) à l'élément de variation de chambre (6).

6. Appareil d'analyse (100) selon la revendication 2 et selon la revendication 5,
**caractérisé en ce que**
l'appareil d'analyse (100) comprend un élément de liaison (4, 11) mécanique,
dans lequel l'élément de liaison (4, 11) mécanique comprend ou met à disposition à la fois l'élément de liaison de soupape et l'élément de liaison de chambre.

7. Appareil d'analyse (100) selon l'une des revendications précédentes,
**caractérisé en ce que**
l'appareil d'analyse (100) comprend un capteur de débit volumique (MP.1, MP.2), dans lequel le capteur de débit volumique (MP.1, MP.2) est configuré pour mesurer une mesure du débit volumique d'un gaz (Pr) à travers le raccordement fluidique d'entrée (31, 15, Sp, 18) dans la chambre de mesure (3), et
dans lequel l'appareil d'analyse (100) est configuré pour déclencher, automatiquement et en fonction du débit volumique mesuré, l'étape
selon laquelle l'unité d'entraînement (7, 4, 11) déplace la soupape (2, 13) en position finale de fermeture en fonction du débit volumique mesuré.

8. Appareil d'analyse (100) selon l'une des revendications précédentes,
**caractérisé en ce que**
la soupape (2, 13) comprend une pièce de fermeture (2), et
l'appareil d'analyse (100) comprend une unité de guidage fluidique (16) entourant la pièce de fermeture (2),
dans lequel un espace intermédiaire (Sp) apparaît entre l'unité de guidage fluidique (16) et la pièce de fermeture (2), et
dans lequel le raccordement fluidique d'entrée (31, 15, Sp, 18)
- passe à travers l'unité de guidage fluidique (16), et
- comprend l'espace intermédiaire (Sp).

9. Appareil d'analyse (100) selon l'une des revendications précédentes,
**caractérisé en ce que**
l'appareil d'analyse (100) est configuré de sorte
qu'un passage de l'unité à chambre d'aspiration (5, 6) dans l'état de volume minimal est provoqué,
que du gaz est acheminé hors de la chambre de mesure (3) à travers le raccordement fluidique d'entrée (31, 15, Sp, 18).

10. Appareil d'analyse (100) selon l'une des revendications précédentes,
**caractérisé en ce que**
l'appareil d'analyse (100) comprend une unité de guidage fluidique d'entrée (1, 16, 40),
dans lequel l'unité d'entrée (30) peut être raccordée, de préférence raccordée de manière amovible, à l'unité de guidage fluidique d'entrée (1, 16, 40),
dans lequel le raccordement fluidique d'entrée (31, 15, Sp, 18) passe à travers l'unité de guidage fluidique d'entrée (1, 16, 40), et
dans lequel l'unité de guidage fluidique d'entrée (1, 16, 40) entoure, de préférence entoure complètement, la soupape (2, 13).

11. Procédé pour l'analyse d'un échantillon de gaz (A) émis, en particulier expiré, par un sujet, à la recherche d'une substance prédéfinie, en particulier d'alcool,
à l'aide d'un appareil d'analyse (100) comprenant
- une unité d'entrée (30),
- une chambre de mesure (3),
- un capteur (12),
- une unité à chambre d'aspiration (5, 6) pouvant passer sélectivement dans un état de volume minimal et dans un état de volume maximal,
- une soupape (2, 13), et
- une unité d'entraînement (7, 4, 11),
dans lequel l'appareil d'analyse (100) met à disposition
- au moins temporairement, un raccordement fluidique d'entrée (31, 15, Sp, 18) entre l'unité d'entrée (30) et la chambre de mesure (3), et
- au moins temporairement, un raccordement fluidique d'aspiration (8) entre l'unité à chambre d'aspiration (5, 6) et la chambre de mesure (3),
dans lequel, avant la mise en oeuvre du procédé, la soupape (2, 13) est dans une position finale de fermeture dans laquelle la soupape (2, 13) interrompt le raccordement fluidique d'entrée (31, 15, Sp, 18), et
dans lequel le procédé comprend les étapes selon lesquelles
- l'échantillon de gaz (A) entre dans l'unité d'entrée (30) et/ou est prélevé par l'unité d'entrée (30),
- l'unité d'entraînement (7, 4, 11) déplace la soupape (2, 13) dans une position finale de libération dans laquelle la soupape (2, 13) libère le raccordement fluidique d'entrée (31, 15, Sp, 18),
- l'unité d'entraînement (7, 4, 11) fait passer l'unité à chambre d'aspiration (5, 6) dans l'état de volume maximal de sorte que du gaz est aspiré, depuis l'unité d'entrée (30) et à travers le raccordement fluidique d'entrée (31, 15, Sp, 18), dans la chambre de mesure (3),
- l'unité d'entraînement (7, 4, 11) fait ensuite passer l'unité à chambre d'aspiration (5, 6) dans l'état de volume minimal de sorte que du gaz est acheminé hors de l'unité à chambre d'aspiration (5, 6), à travers le raccordement fluidique d'aspiration (8) et dans la chambre de mesure (3) et du gaz est ainsi expulsé de la chambre de mesure (3), et
- l'unité d'entraînement (7, 4, 11) déplace à nouveau la soupape (2, 13) en position finale de fermeture,
dans lequel le procédé comprend l'étape supplémentaire selon laquelle le capteur (12) mesure une mesure de la concentration et/ou de la quantité de la substance dans le gaz (Pr) se trouvant dans la chambre de mesure (3),
dans lequel
soit
- l'étape consistant à déplacer la soupape (2, 13) en position finale de libération et l'étape consistant à faire passer l'unité à chambre d'aspiration (5, 6) dans l'état de volume minimal sont mises en œuvre simultanément, et
- l'étape consistant à déplacer à nouveau la soupape (2, 13) en position finale de fermeture et l'étape consistant à faire passer l'unité à chambre d'aspiration (5, 6) dans l'état de volume maximal sont effectuées simultanément,
soit
- l'étape consistant à déplacer la soupape (2, 13) en position finale de fermeture et l'étape consistant à faire passer l'unité à chambre d'aspiration (5, 6) dans l'état de volume minimal sont mises en œuvre simultanément, et
- l'étape consistant à déplacer à nouveau la soupape (2, 13) en position finale de libération et l'étape consistant à faire passer l'unité à chambre d'aspiration (5, 6) dans l'état de volume maximal sont effectuées simultanément.

12. Procédé selon la revendication 11,
**caractérisé en ce que**
l'évènement selon lequel l'entrée de l'échantillon de gaz (A) dans l'unité d'entrée (30) débute est détecté automatiquement, et
l'étape consistant à déplacer la soupape (2, 13) en position finale de libération débute lorsque
- une période prédéfinie s'est écoulée depuis le début de l'entrée de l'échantillon de gaz (A), et/ou
- un évènement d'ouverture s'est produit après le début de l'entrée de l'échantillon de gaz (A),
dans lequel l'évènement d'ouverture dépend d'une mesure du volume et/ou de la quantité de l'échantillon de gaz (A) entré jusqu'à présent dans l'unité d'entrée (30).

13. Procédé selon la revendication 11 ou la revendication 12,
**caractérisé en ce que**
une mesure de la quantité de gaz qui s'est écoulée jusqu'à présent dans la chambre de mesure (3) après le début de l'étape consistant à déplacer la soupape (2, 13) en position finale de libération est mesurée au moins une fois, et
lorsque la quantité mesurée a atteint une limite de quantité prédéfinie, l'étape consistant à déplacer à nouveau la soupape (2, 13) en position finale de fermeture est automatiquement déclenchée.

14. Procédé selon l'une des revendications 11 à 13,
**caractérisé en ce que**
avant la mise en œuvre du procédé, l'unité à chambre d'aspiration (5, 6) est dans l'état de volume maximal, et
l'étape consistant à faire passer l'unité à chambre d'aspiration (5, 6) dans l'état de volume minimal
est mise en œuvre avant l'étape consistant à faire passer l'unité à chambre d'aspiration (5, 6) dans l'état de volume maximal.

15. Procédé selon l'une des revendications 11 à 13,
**caractérisé en ce que**
avant la mise en œuvre du procédé, l'unité à chambre d'aspiration (5, 6) est dans l'état de volume minimal, et
l'étape consistant à faire passer l'unité à chambre d'aspiration (5, 6) dans l'état de volume maximal
est mise en œuvre avant l'étape consistant à faire passer l'unité à chambre d'aspiration (5, 6) dans l'état de volume minimal.

16. Procédé selon l'une des revendications 11 à 15,
**caractérisé en ce que**
l'unité d'entraînement (7, 4, 11) provoque les étapes consistant à
- déplacer la soupape dans une position finale, et
- faire passer l'unité à chambre d'aspiration (5, 6) dans l'état de volume minimal ou maximal
à l'aide d'un accouplement mécanique de l'unité d'entraînement (7, 4, 11) à la soupape (2, 13) et à l'unité à chambre d'aspiration (5, 6).
